# EUROPEAN PATENT SPECIFICATION

(11) **EP 2 329 259 B1**
(45) Date of publication and mention of the grant of the patent: **20.04.2016**
(21) Application number: 09810643.8
(22) Date of filing: 28.08.2009
(51) Int. Cl.: G01N 33/48, C12Q 1/68

(54) **MARKERS AND METHODS FOR ASSESSING AND TREATING ULCERATIVE COLITIS AND RELATED DISORDERS USING A 20 GENE PANEL**
MARKER UND VERFAHREN ZUR BEURTEILUNG UND BEHANDLUNG VON COLITIS ULCEROSA SOWIE VERWANDTEN ERKRANKUNGEN MIT EINEM 20-GEN-PANEL
MARQUEURS ET PROCÉDÉS POUR ÉVALUER ET POUR TRAITER UNE RECTO-COLITE HÉMORRAGIQUE ET DES TROUBLES ASSOCIÉS À L'AIDE D'UN ENSEMBLE DE 20 GÈNES

(30) Priority: 29.08.2008 US 92966 P
(43) Date of publication of application: 08.06.2011
(73) Proprietor: Janssen Biotech, Inc., Horsham, PA 19044 (US)
(72) Inventor: LI, Xilin, Radnor, PA 19087 (US); BARIBAUD, Frederic, Radnor, PA 19087 (US)
(74) Representative: Goodfellow, Hugh Robin
(86) International application number: PCT/US2009/055323
(87) International publication number: WO 2010/025340

(56) References cited:
- WO-A2-2007/103823
- WO-A2-2008/028044
- WO-A2-2008/028044
- WO-A2-2008/137383
- WO-A2-2010/044952
- US-A1- 2004 077 020
- US-A1- 2006 134 663
- SCHENK MIRJAM ET AL: "TREM-1-expressing intestinal macrophages crucially amplify chronic inflammation in experimental colitis and inflammatory bowel diseases", JOURNAL OF CLINICAL INVESTIGATION, vol. 117, no. 10, October 2007 (2007-10), pages 3097-3106, XP002672633, ISSN: 0021-9738

### SEQUENCE LISTING

<110> CENTOCOR ORTHO BIOTECH INC.
<120> MARKERS AND METHODS FOR ASSESSING AND TREATING ULCERATIVE COLITIS AND RELATED DISORDERS USING A 20 GENE PANEL
<130> CEN5230PCT
<140> T0 BE ASSIGNED
   <141> 2009-08-28
<150> 61/092966
   <151> 2008-08-29
<160> 20
<170> FastSEQ for Windows Version 4.0
<210> 1
   <211> 553
   <212> DNA
   <213> Homo sapiens
<400> 1
   gatctacttg tgacttgttg gccttcttcc cacatctgcc tcagactggg gggggctcag 60
   ctcctcgggt gatatctagc ctgcttgtga gctctagcag ggataaggag agctgagatt 120
   ggagggaatt gtgttgctcc tggaggaagc ccaggcatca ttaaacaagc cagtaggtca 180
   cctggcttcc gtggaccaat tcatctttca gacaagcttt agagaaatgg actcagggaa 240
   gagactcaca tgctttggtt agtatctgtg tttccggtgg gtgtaatagg ggattagccc 300
   cagaagggac tgagctaaac agtgttatta tgggaaagga aatggcattg ctgctttcaa 360
   ccagcgacta atgcaatcca ttcctctctt gtttatagta atctaagggt tgagcagtta 420
   aaacggcttc aggatagaaa gctgtttccc acctgtttcg ttttaccatt aaaagggaaa 480
   cgtgcctctg ccccacgggt agagggggtg cacgttcctc ctggttcctt cgcttgtgtt 540
   tctgtactta cca                                                    553
<210> 2
   <211> 467
   <212> DNA
   <213> Homo sapiens
<400> 2
   ccatgatcat ggtttactgc gcgtccgaat ggtcaacctt caagtggaag attctggact 60
   gtatcagtgt gtgatctacc agcctcccaa ggagcctcac atgctgttcg atcgcatccg 120
   cttggtggtg accaagggtt tttcagggac ccctggctcc aatgagaatt ctacccagaa 180
   tgtgtataag attcctccta ccaccactaa ggccttgtgc ccactctata ccagccccag 240
   aactgtgacc caagctccac ccaagtcaac tgccgatgtc tccactcctg actctgaaat 300
   caaccttaca aatgtgacag atatcatcag ggttccggtg ttcaacattg tcattctcct 360
   ggctggtgga ttcctgagta agagcctggt cttctctgtc ctgtttgctg tcacgctgag 420
   gtcatttgta ccctaggccc acgaacccac gagaatgtcc tctgact               467
<210> 3
   <211> 307
   <212> DNA
   <213> Homo sapiens
<220>
   <221> unknown
   <222> (60)
   <223> wherein n can be represented by a, c, t, or g
<400> 3
   agttagaggc tgtacaaggc ccccactgcc tgtcggttgc ttggattccc tgacgtaagn 60
   tggatattaa aaatctgtaa atcaggacag gtggtgcaaa tggcgctggg aggtgtacac 120
   ggaggtctct gtaaaagcag acccacctcc cagcgccggg aagcccgtct tgggtcctcg 180
   ctgctggctg ctccccctgg tggtggatcc tggaattttc tcacgcagga gccattgctc 240
   tcctagaggg ggtctcagaa actgcgaggc cagttccttg gagggacatg actaatttat 300
   cgatttt 307
<210> 4
   <211> 503
   <212> DNA
   <213> Homo sapiens
<220>
   <221> unknown
   <222> (419)
   <223> wherein n can be represented by a, c, t, or g
<400> 4
   gtgctcggcc tgatggagac tgtgtgcagc cccttcacgg ccgccagacg tctgcgggac 60
   caggaggcag ccgtggcgga gctgcaggcc gccctggagc gacaggctct ccagaagcaa 120
   gccctgcagg agaaaggcaa gcagcaggac acggtcctcg gcggccgggc cctgtccaac 180
   cggcagcacg cctcctagga actgtgggag accagcggag tgggagggag acgcagtaga 240
   cagagacaga ccgagaagga agggagagac agagggggcg cgcgcacagg agcctgactc 300
   cgctgggaga gtgcaggagc acgtgctgtt ttttatttgg acttaacttc agagaaaccg 360
   ctgacatcta gaactgacct accacaagca tccaccaaag gagtttggga ttgagtttnt 420
   gctgctgtgc agcactgcat tgtcatgaca tttccaacac tgtgtgaatt atctaaatgc 480
   gtctaccatt ttgcactagg gag 503
<210> 5
   <211> 542
   <212> DNA
   <213> Homo sapiens
<400> 5
   tttcctcttt ttgactacag taactattcc aaatggggac acatactgta ctttcaactt 60
   tgcatcctgg ggtggcaccc ctgaggagag gctgaaggtg gccattacca tgctgacagc 120
   cagagggatt atccggtttg tcattggctt tagcttgccg atgtccattg ttgccatctg 180
   ctatgggctc attgcagcca agatccacaa aaagggcatg attaaatcca gccgtccctt 240
   acgggtcctc actgctgtgg tggcttcttt cttcatctgt tggtttccct ttcaactggt 300
   tgcccttctg ggcaccgtct ggctcaaaga gatgttgttc tatggcaagt acaaaatcat 360
   tgacatcctg gttaacccaa cgagctccct ggccttcttc aacagctgcc tcaaccccat 420
   gctttacgtc tttgtgggcc aagacttccg agagagactg atccactccc tgcccaccag 480
   tctggagagg gccctgtctg aggactcagc cccaactaat gacacggctg ccaattctgc 540
   tt 542
<210> 6
   <211> 229
   <212> DNA
   <213> Homo sapiens
<400> 6
   cccacaaccc gccaggattt aatcttttat ggattttttt ttcagtttat agtttccagg 60
   aggaaagtct tgtaacgctt tactttttct cttctttttt taaagtaaag tgagtgtcag 120
   tagatgtgtt ttttacagtc tggattttaa aaaaagtggg gcaggagaga gggaaccagg 180
   gaaagggctg tttagaaata cagtttgtgg ctctgagcga tgccaccgg             229
<210> 7
   <211> 294
   <212> DNA
   <213> Homo sapiens
<220>
   <221> unknown
   <222> (45) (53) (62) (77) (84)
<400> 7
   gaatatgcag caatggaaag tatcagtatc catacaaaga ttttncaaga aancgaatgg 60
   gntgagccaa gtagctngag attnagaatg agttcataga ctgtccaaga tctttatccc 120
   aaaaacattt ggattgttta ttttcagtat ttatcctctt tccctactac ctggatactc 180
   caaatttgca tatacttgta atattaatat acagttcctt tgcaatattt attttgttgc 240
   ttcttagaga aacccaacta aatgagttta aaaaatctgt gaaagggctg ggca       294
<210> 8
   <211> 432
   <212> DNA
   <213> Homo sapiens
<400> 8
   aagtgcttca agtattagtt gtcatattaa tccaagagaa aattagatct gagactacct 60
   cgaggagtaa acgaacaact tctttaaacc aatactagtc aaaatgcagt ccacggacca 120
   atgctggttc atgccctatt tgatccaagt ccacattaag tacagaaatg aagagtaagc 180
   atttagaaac ttggggcaat ttgacattgc ctcaacatcc aagtttcagt gtcctttata 240
   aactattgat ccatgacaga tcatgaaact ttctaaaaat tgatcctttt atcacatagt 300
   ctttcaaaat attatttgct tctttgaagc tttggataga aacttgataa tgtgaagttc 360
   tctgctatgt ggacctggtg tgcttatctt ctttgttgct gatttgagag ctgagctatg 420
   tactatatct gg                                                     432
<210> 9
   <211> 569
   <212> DNA
   <213> Homo sapiens
<220>
   <221> unknown
   <222> (120) (180) (395) (413) (416) (417)
<400> 9
   tggggcgcct atgatggaga ccctcagccc ctagggactc catcttttga ccttgaagtc 60
   tcgctcaaac attcccctaa aagaacgggg gtgagggaga atggaaggca aaaagagngn 120
   aaaaagcaaa taaacttcgg aatcggacaa cttaaagtct cgatatgagc ctcggattgn 180
   attcgaactc ctgggttcta gacccagctc tgctactaac ttgtctgacc ttccccattt 240
   ataaactgtt gtgggtgaga ggtgggacta ggtgaactct gagagccctg cactcggcgc 300
   ggacgaggga tttaggggaa agtgtgctgt attccccgcg agccttcccc agcggccccg 360
   cctccccggg tgaatccccg gcggccagcg cagcncaggc agcgatcctg gcngcnnccg 420
   gggccggcag cttcctggaa agttacttct tgttggagcc ggcaataggc aaagtgtgtt 480
   ctgtgaaatc cgcagagccg agattgacta cgttccggga atgagagggc tgtgtcattc 540
   ccctcattgc ctgctgcatt gacggcgta                                   569
<210> 10
   <211> 456
   <212> DNA
   <213> Homo sapiens
<220>
   <221> unknown
   <222> (38) (52) (54) (56) (64) (69) (79) (82) (86) (88) (95) (99) (104) (116)
   <223> wherein n can be represented by a, c, t, or g
<400> 10
   actgcagcaa actttctagc atctgatatt ggataagnat agcttgtgct anangntgga 60
   gatnaatcng gtctgctgnc tngcancntt agagnctgna tctnatggtt ggtgtnagga 120
   tgttgttgac agttctgaaa gttagccatc aattcctgtg cagggtggag tcagacccag 180
   tgacttcctt ttcaatgtca gcaagagttt tctcatgcct gctttggtca ctttctcttg 240
   gaaacttcac gcatttgact tgcagcttct tgacccgagg aatcaactga gctcccagtg 300
   ctggctacct tgggcaaata aaatctgtgg ttgagagagt tctctttgct gtgccacagt 360
   ccctgtgatg tgccaaatgg caccagcatt tgcagcaaag ctcgttaaat ctttttggaa 420
   tgggcaaata ttttttcaac ttgagccctg ctgagc                           456
<210> 11
   <211> 550
   <212> DNA
   <213> Homo sapiens
<220>
   <221> unknown
   <222> (51) (123)...(139) (272) (273) (274) (301)...(369)
   <223> wherein n can be represented by a, c, t, or g
<400> 11
   aatgtggact caatccctag ggctggcaga aagggaacag aaaggttttt nagtacggct 60
   atagcctgga ctttcctgtt gtctacacca atgcccaact gcctgcctta gggtagtgct 120
   aannnnnnnn nnnnnnnnnc agccaggaca gtcagctctc tcctttcagg gccaatcccc 180
   agcccttttg ttgagccagg cctctctcac ctctcctact cacttaaagc ccgcctgaca 240
   gaaaccacgg ccacatttgg ttctaagaaa cnnncctctg tcattcgctc ccacattctg 300
   nnnnnnnnnn nnnnnnnnnn nnnnnnnnnn nnnnnnnnnn nnnnnnnnnn nnnnnnnnnn 360
   nnnnnnnnna agggggcaag aagtagcagt gtctgtaaaa gagcctagtt tttaatagct 420
   atggaatcaa ttcaatttgg actggtgtgc tctctttaaa tcaagtcctt taattaagac 480
   tgaaaatata taagctcaga ttatttaaat gggaatattt ataaatgagc aaatatcata 540
   ctgttcaatg                                                        550
<210> 12
   <211> 519
   <212> DNA
   <213> Homo sapiens
<220>
   <221> unknown
   <222> (325)...(331)
   <223> wherein n can be represented by a, c, t, or g
<400> 12
   aacagtacag tcctcaccct gatgaccttg accccagagg ggtcggagct acacatcatc 60
   ctgggcctgt tcggcctcct gctgttgctc acctgcctct gtggaactgc ctggctctgt 120
   tgcagcccca acaggaagaa tcccctctgg ccaagtgtcc cagacccagc tcacagcagc 180
   ctgggctcct gggtgcccac aatcatggag gaggatgcct tccagctgcc cggccttggc 240
   acgccaccca tcaccaagct cacagtgctg gaggaggatg aaaagaagcc ggtgccctgg 300
   gagtcccata acagctcaga gaccnnnnnn ntccccactc tggtccagac ctatgtgctc 360
   cagggggacc caagagcagt ttccacccag ccccaatccc agtctggcac cagcgatcag 420
   gctgggcctc ccaggcgatc tgcatacttt aaggaccaga tcatgctcca tccagcccca 480
   cccaatggcc ttttgtgctt gtttcctata acttcagta                        519
<210> 13
   <211> 462
   <212> DNA
   <213> Homo sapiens
<400> 13
   gtttcttaca gtgagcggga tgcctactgg gtggagaacc ttatggtcca ggagctggag 60
   aacttcaatc cccccttcaa gttgtgtctt cataagcggg acttcattcc tggcaagtgg 120
   atcattgaca atatcattga ctccattgaa aagagccaca aaactgtctt tgtgctttct 180
   gaaaactttg tgaagagtga gtggtgcaag tatgaactgg acttctccca tttccgtctt 240
   tttgaagaga acaatgatgc tgccattctc attcttctgg agcccattga gaaaaaagcc 300
   attccccagc gcttctgcaa gctgcggaag ataatgaaca ccaagaccta cctggagtgg 360
   cccatggacg aggctcagcg ggaaggattt tgggtaaatc tgagagctgc gataaagtcc 420
   taggttccca tatttaagac cagtctttgt ctagttggga tc                    462
<210> 14
   <211> 340
   <212> DNA
   <213> Homo sapiens
<400> 14
   attatgcttt ctattttgag atcattgcaa actcaacaca attgtaagta atgatacaga 60
   gggatcttgt gtacccttca cccagcctcc cccaatggca acatcttgca aaactacaat 120
   gtagtctcat aaccaggata ttgacattga tacagtgaag atacaggaca ttctcatcac 180
   cacagggatc cccaggatgc ccacttccct ccacccccac accccagccg tgtccctaac 240
   ccctggcaac caggaatcca ctctccattt ctataatgtt gtcatttcaa gaatgttatt 300
   caatggaatc atatagtatg taacctgttt tgagcttaaa                        340
<210> 15
   <211> 418
   <212> DNA
   <213> Homo sapiens
<400> 15
   gagggcaacc accagtttgc taagtacaaa tcattcaagg tggctgacga ggcagagaag 60
   tacaagctgg tactgggagc ctttgtcggg ggcagtgcgg gtaattctct aacgggccac 120
   aacaacaact tcttctccac caaagaccaa gacaatgatg tgagttcttc gaattgtgct 180
   gagaagttcc agggagcctg gtggtacgcc gactgtcatg cttcaaacct caatggtctc 240
   tacctcatgg gaccccatga gagctatgcc aatggtatca actggagtgc ggcgaagggg 300
   tacaaatata gctacaaggt gtcagagatg aaggtgcggc ccgcctagac gggccaggac 360
   ccctccacat gcacctgcta gtggggaggc cacacccaca agcgctgcgt cgtggaag 418
<210> 16
   <211> 349
   <212> DNA
   <213> Homo sapiens
<400> 16
   ataccgctgt tactactaca gccctgcagg ctggtcagag cccagcgacc ccctggagct 60
   ggtggtgaca ggattctaca acaaacccac cctctcagcc ctgcccagtc ctgtggtgac 120
   ctcaggagag aacgtgaccc tccagtgtgg ctcacggctg agattcgaca ggttcattct 180
   gactgaggaa ggagaccaca agctctcctg gaccttggac tcacagctga cccccagtgg 240
   gcagttccag gccctgttcc ctgtgggccc tgtgaccccc agccacaggt ggatgctcag 300
   atgctatggc tctcgcaggc atatcctgca ggtatggtca gaacccagt 349
<210> 17
   <211> 479
   <212> DNA
   <213> Homo sapiens
<400> 17
   gtgcagtcat caccaagcag aatgggaagc tgtgcttggt gattcaggta gccaatatga 60
   ggaagagcct cttgattcag tgccagctct ctggcaagct cctgcagacc cacgtcacca 120
   aggaggggga gcggattctc ctcaaccaag ccactgtcaa attccacgtg gactcctcct 180
   ctgagggccc cttcctcatt ctgcccatga cattctacca tgtgctggat gagacgagcc 240
   ccctgagaga cctcacaccc caaaacctaa aggagaagga gtttgagctt gtggtcctcc 300
   tcaatgccac tgtggaatcc accagcgctg tctgccagag ccgaacatct tatatcccag 360
   aggaaatcta ctggggtttt gagtttgtgc ctgtggtatc tctctccaaa aatggaaaat 420
   atgtggctga tttcagtcag tttgaacaga ttcggaaaag cccagattgc acattttac 479
<210> 18
   <211> 285
   <212> DNA
   <213> Homo sapiens
<400> 18
   aagtgctcag accttgtcta gttatttata aactgtatgt acctccctca cttctctcct 60
   atcactgctt tcctactctc cttttatctc actctagtcc aggtgccaag aatttccctt 120
   ctaccctcta ttctcttgtg tctgtaagtt acaaagtcag gaaaagtctt ggctggaccc 180
   ctttcctgct gggtggatgc agtggtccag gactggggtc tgggcccagg tttgagggag 240
   aagtttgcag agcacttccc acctctctga atagtgtgta tgtgt 285
<210> 19
   <211> 478
   <212> DNA
   <213> Homo sapiens
<400> 19
   ccccctggac atcctgatca caggacagtt ctatgacaga ccctctctct cggtgcagcc 60
   ggtccccaca gtagccccag gaaagaacgt gaccctgctg tgtcagtcac gggggcagtt 120
   ccacactttc cttctgacca aggagggggc aggccatccc ccactgcatc tgagatcaga 180
   gcaccaagct cagcagaacc aggctgaatt ccgcatgggt cctgtgacct cagcccacgt 240
   ggggacctac agatgctaca gctcactcag ctccaacccc tacctgctgt ctctccccag 300
   tgaccccctg gagctcgtgg tctcagaagc agctgagacc ctcagcccat cacaaaacaa 360
   gacagactcc acgactacat ccctaggcca acacccccag gattacacag tggagaatct 420
   catccgcatg ggtgtggctg gcttggtcct ggtggtcctc gggattctgc tatttgag   478
<210> 20
   <211> 437
   <212> DNA
   <213> Homo sapiens
<220>
   <221> unknown
   <222> (186) (199) (205) (217) (338) (353) (375) (376) (399)
<400> 20
   ggatgccatg aatcgagact gtggaattcc actcagtcat ttgcaggttg atggaggaat 60
   gaccagcaac aaaattctta tgcagctaca agcagacatt ctgtatattc cagtagtgaa 120
   gcccttgatg cccgaaacca ctgcactggg tgctgccatg gcggcagggg ctgcagaagg 180
   agtcgncgta tggagtctng aaccngagga tttgtcngcc gtcacgatgg agcggtttga 240
   acctcagatt aatgctgagg aaagtgaaat tcgttattct acatggaaga aagctgtgat 300
   gaagtcaatg ggttgggtta caactcaatc tccagaangt ggtgacccta gtntcttctg 360
   tagtctgccc ttggnntttt ttatagtgag tagcatggna atgttaatcg gagcaaggta 420
   catctcaggt attccat                                                437

## Description

The invention relates to the identification of expression profiles and the nucleic acids indicative of gastrointestinal-related disorders, such as ulcerative colitis, and to the use of such expression profiles and nucleic acids in diagnosis of ulcerative colitis and related diseases. The invention further relates to methods for identifying, using, and testing candidate agents and/or targets which modulate ulcerative colitis.

### BACKGROUND OF THE INVENTION

Ulcerative colitis (UC) is a multifactorial autoimmune disease with a complex pathogenesis involving unidentified genetic, microbial, and environmental factors. Recent studies using microarray analysis of inflamed colonoscopic tissue biopsy vs. non-inflamed biopsy samples from UC patients revealed dysregulation of a few inflammatory cytokines, however, the etiology, pathogenesis, and role of tumor necrosis factor-alpha (TNFα) in UC is still poorly understood. TNFα is a critical proinflammatory cytokine in Crohn's disease as demonstrated by the therapeutic effect of infliximab on the induction and maintenance of clinical remission, closure of enterocutaneous, perianal, and rectovaginal fistulas, maintenance of fistula closure, and steroid tapering in Crohn's disease patients. However, the evidence to support a role of TNFα in the pathogenesis of UC has been controversial despite the fact that it is also found at increased levels in the blood, colonic tissue, and stools of UC patients. A clinical study (ACT-1) by Rutgeerts et al. showed that infliximab is effective when administered at weeks 0, 2, 6 and every 8 weeks thereafter in achieving clinical response and remission in patients with moderate-to-severe active UC despite the use of conventional therapy supporting a critical pathogenic role of TNFα in UC.

Microarray technology is a powerful tool since it enables analysis of the expression of thousands of genes simultaneously and can also be automated allowing for a high-throughput format. In diseases associated with complex host functions, such as those known as immune mediated inflammatory diseases, such as UC, microarray results can provide a gene expression profile that can be of utility in designing new approaches to disease diagnosis and management. These approaches also serve to identify novel genes and annotating genes of unknown function heretofore unassociated with the disease or condition. Accordingly, there is a need to identify and characterize new gene markers useful in developing methods for diagnosing and treating autoimmune disorders, such as UC and Crohn's disease, as well as other diseases and conditions and how a patient would respond to a therapeutic intervention.

Gene expression can be modulated in several different ways, including by the use of siRNAs, shRNAs, antisense molecules and DNAzymes. SiRNAs and shRNAs both work via the RNAi pathway and have been successfully used to suppress the expression of genes. RNAi was first discovered in worms and the phenomenon of gene silencing related to dsRNA was first reported in plants by Fire and Mello and is thought to be a way for plant cells to combat infection with RNA viruses. In this pathway, the long dsRNA viral product is processed into smaller fragments of 21-25 bp in length by a DICER-like enzyme and then the double-stranded molecule is unwound and loaded into the RNA induced silencing complex (RISC). A similar pathway has been identified in mammalian cells with the notable difference that the dsRNA molecules must be smaller than 30 bp in length in order to avoid the induction of the so-called interferon response, which is not gene specific and leads to the global shut down of protein synthesis in the cell.

Synthetic siRNAs have been successfully designed to selectively target a single gene and can be delivered to cells in vitro or in vivo. ShRNAs are the DNA equivalents of siRNA molecules and have the advantage of being incorporated into a cells' genome where they are replicated during every mitotic cycle.

DNAzymes have also been used to modulate gene expression. DNAzymes are catalytic DNA molecules that cleave single-stranded RNA. They are highly selective for the target RNA sequence and as such can be used to down-regulate specific genes through targeting of the messenger RNA.

US2004/077020 describes a method for testing therapeutic compounds by detecting changes in expression levels of different genes. WO2008/028044 refers to a method testing therapies for effectivenesss using a 66-member gene panel. WO2007/103823 describes a method for predicting a suitable therapy for patients suffering from gastrointestinal cancer by determining the expression of certain marker genes.

Accordingly, there is a need to identify and characterize new gene markers useful in developing methods for diagnosing and treating autoimmune disorders, such as UC and Crohn's disease, as well as other diseases and conditions.

### SUMMARY OF THE INVENTION

The invention provides a method for predicting the suitability of treatment with a target therapy for a gastrointestinal-related disorder in a subject, wherein the subject is a patient providing the sample prior to administration of the therapy, and wherein the therapy is an anti-TNFα antibody, comprising:
a) preparing a sample of nucleic acids from a specimen obtained from the subject;
b) contacting the sample with a panel of nucleic acid segments consisting of SEQ ID NOS:1-20 to detect levels of the panel segments;
c) evaluating the sample against a reference standard of a responder to the therapy to determine the relative expression levels of all members from the group consisting of the nucleotide sequences corresponding to SEQ ID NOS:1-20 compared to the reference standard; and
d) correlating the relative expression levels of the sample and the reference standard with the suitability of treatment with the target therapy for the gastrointestinal-related disorder.

The invention also provides a reagent for testing the suitability of an anti-TNFα antibody for a gastrointestinal-related disorder in a cell or subject prior to administration of the anti-TNFα antibody, comprising oligonucleotides comprising at least 15 nucleotides comprising or complementary to a nucleotide sequence of each of the nucleotide sequences corresponding to SEQ ID NOS: 1-20, a polypeptide encoded by at least a portion of each of the nucleotide sequences corresponding to SEQ ID NOS: 1-20, and a ligand for the polypeptide encoded by each one of the nucleotide sequences corresponding to SEQ ID NOS: 1-20.

The invention also provides method of testing the suitability of an anti-TNFα antibody for a gastrointestinal-related disorder in a patient sample from a patient prior to administration of the anti-TNFα antibody, comprising contacting the patient sample with the reagent of the invention and comparing the levels of at least a portion of each of the genes or proteins of the nucleotide sequences corresponding to SEQ ID NOS: 1-20 to a reference standard, wherein the reference standard is of a responder to the anti-TNFα antibody.

The invention also provides a method of testing the effectiveness of an anti-TNFα antibody for ulcerative colitis, comprising:
a) contacting a sample from a patient being treated for ulcerative colitis with the reagent of the invention;
b) measuring levels of the 20 members; and
c) correlating the levels of the 20 members with the effectiveness of the anti-TNFα antibody by comparing the levels to the levels in a reference standard, wherein the reference standard is of a responder to the anti-TNFα antibody.

### SUMMARY OF THE INVENTION

The present disclosure relates to a method of diagnosing and/or treating UC and/or related diseases or disorders and predicting the suitability of candidate agents for treatment. The present disclosure includes the discovery of panels of genes, one of 20 genes and one of five genes, that have modified expression levels in patients responsive to treatment for UC (effective in reducing the symptoms of UC) versus patients nonresponsive to treatment. The modified expression levels constitute a profile that can serve as a biomarker profile predictive of a patient's responsiveness to treatment.

In a particular embodiment, the present disclosure comprises a method of predicting the suitability of a treatment for UC based on the pattern of gene expression of one or more of the 20 genes which constitute the profile prior to treatment. One or more of these genes may be from a category of genes, such as those involved in defense response, immune response, signal transduction, and pathogen response as shown below and in Figure 1, and the like. In a typical embodiment, the cell specimen expresses at least two expression profile genes. The profile genes may show an increase or decrease.

In addition, the present invention comprises a method of identifying subjects with UC and/or related diseases or disorders that are candidates for treatment with a particular therapeutic agent by evaluating their expression profile of one or more genes of the 20- or 5-gene panel.

In one embodiment, the UC-related gene profile is used to create an array-based method for prognostic or diagnostic purposes, the method comprising:
(a) preparing a representative mixture of nucleic acids from a specimen obtained from a patient and causing said sample nucleic acids in the mixture to be labeled with a detectable marker;
(b) contacting a sample with an array comprising a plurality of nucleic acid segments, wherein each nucleic acid segment is immobilized to a discrete and known address on a substrate surface wherein the panel of UC-related biomarkers is identified as a feature of the array by address, the array further comprises at least one calibration nucleic acid at a known address on the substrate, and contacting is performed under conditions in which a sample nucleic acid specifically may bind to the nucleic acid segment immobilized on the arrays;
(c) performing a statistical comparison of all test samples from treated patients and a reference standard; and
(d) comparing the pattern of intensity changes in features for the test sample to the pattern of intensity changes for those features which are members of the UC-related gene profile with historical patterns for samples taken from patients responsive to treatment with an anti-TNF antibody.

Optionally, statistical analysis is performed on the changes in levels of members of the gene panel to evaluate the significance of these changes and to identify which members are meaningful members of the panel.

In an alternative embodiment, the present disclosure comprises a kit for predicting the suitability of candidate agents for treating UC and/or related diseases or disorders based on the pattern of gene expression.

### DESCRIPTION OF THE FIGURES

Figure 1 shows the distribution of biological processes for the members of the gene panels.
Figure 2 shows the Infliximab responder/non-responder expression profile of the 5 probe set classifier in a dot plot representation comparing infliximab responders (R) to nonresponders (NR). The normalized intensities of each sample are shown (black circle). The median intensity, the 75th and 25th percentile and the minimum and maximum values for each responder and nonresponder population for each of the 5 genes are also shown.

### DETAILED DESCRIPTION OF THE INVENTION

### Definitions

The following definitions are set forth to illustrate and define the meaning and scope of various terms used to describe the invention herein.

An "activity," a biological activity, and a functional activity of a polypeptide refers to an activity exerted by a gene of the UC-related gene panel in response to its specific interaction with another protein or molecule as determined in vivo, in situ, or in vitro, according to standard techniques. Such activities can be a direct activity, such as an association with or an enzymatic activity on a second protein, or an indirect activity, such as a cellular process mediated by interaction of the protein with a second protein or a series of interactions as in intracellular signaling or the coagulation cascade.

An "antibody" includes any polypeptide or peptide containing molecule that comprises at least a portion of an immunoglobulin molecule, such as but not limited to, at least one complementarity determining region (CDR) of a heavy or light chain or a ligand binding portion thereof, a heavy chain or light chain variable region, a heavy chain or light chain constant region, a framework region, or any portion, fragment or variant thereof. The term "antibody" is further intended to encompass antibodies, digestion fragments, specified portions and variants thereof, including antibody mimetics or comprising portions of antibodies that mimic the structure and/or function of an antibody or specified fragment or portion thereof, including single chain antibodies and fragments thereof. For example, antibody fragments include, but are not limited to, Fab (e.g., by papain digestion), Fab' (e.g., by pepsin digestion and partial reduction) and F(ab')2 (e.g., by pepsin digestion), facb (e.g., by plasmin digestion), pFc' (e.g., by pepsin or plasmin digestion), Fd (e.g., by pepsin digestion, partial reduction and reaggregation), Fv or scFv (e.g., by molecular biology techniques) fragments, and single domain antibodies (e.g., V_{H} or V_{L}), are encompassed by the invention (see, e.g., Colligan, et al., eds., Current Protocols in Immunology, John Wiley & Sons, Inc., NY (1994-2001); Colligan et al., Current Protocols in Polypeptide Science, John Wiley & Sons, NY (1997-2001)).

The terms "array" or "microarray" or "biochip" or "chip" as used herein refer to articles of manufacture or devices comprising a plurality of immobilized target elements, each target element comprising a "clone," "feature," "spot" or defined area comprising a particular composition, such as a biological molecule, e.g., a nucleic acid molecule or polypeptide, immobilized to a solid surface, as discussed in further detail, below.

"Complement of" or "complementary to" a nucleic acid sequence of the invention refers to a polynucleotide molecule having a complementary base sequence and reverse orientation as compared to a first polynucleotide.

"Identity," as known in the art, is a relationship between two or more polypeptide sequences or two or more polynucleotide sequences, as determined by comparing the sequences. In the art, "identity" also means the degree of sequence relatedness between polypeptide or polynucleotide sequences, as determined by the match between strings of such sequences. "Identity" and "similarity" can be readily calculated by known methods, including, but not limited to, those described in Computational Molecular Biology, Lesk, A. M., ed., Oxford University Press, New York, 1988; Biocomputing:Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; and Carillo, H., and Lipman, D., Siam J. Applied Math., 48:1073 (1988). In addition, values for percentage identity can be obtained from amino acid and nucleotide sequence alignments generated using the default settings for the AlignX component of Vector NTI Suite 8.0 (Informax, Frederick, MD).

The terms "specifically hybridize to," "hybridizing specifically to," "specific hybridization" and "selectively hybridize to," as used herein refer to the binding, duplexing, or hybridizing of a nucleic acid molecule preferentially to a particular nucleotide sequence under stringent conditions. The term "stringent conditions" refers to conditions under which a probe will hybridize preferentially to its target subsequence; and to a lesser extent to, or not at all to, other sequences. A "stringent hybridization" and "stringent hybridization wash conditions" in the context of nucleic acid hybridization (e.g., as in array, Southern or Northern hybridizations) are sequence dependent, and are different under different environmental parameters. Alternative hybridization conditions that can be used to practice the invention are described in detail, below. In alternative aspects, the hybridization and/or wash conditions are carried out under moderate conditions, stringent conditions and very stringent conditions, as described in further detail, below. Alternative wash conditions are also used in different aspects, as described in further detail, herein.

The phrases "labeled biological molecule" or "labeled with a detectable composition" or "labeled with a detectable moiety" as used herein refer to a biological molecule, e.g., a nucleic acid, comprising a detectable composition, i.e., a label, as described in detail, below. The label can also be another biological molecule, as a nucleic acid, e.g., a nucleic acid in the form of a stem-loop structure as a "molecular beacon," as described below. This includes incorporation of labeled bases (or, bases which can bind to a detectable label) into the nucleic acid by, e.g., nick translation, random primer extension, amplification with degenerate primers, and the like. Any label can be used, e.g., chemiluminescent labels, radiolabels, enzymatic labels and the like. The label can be detectable by any means, e.g., visual, spectroscopic, photochemical, biochemical, immunochemical, physical, chemical and/or chemiluminescent detection. The invention can use arrays comprising immobilized nucleic acids comprising detectable labels.

The term "nucleic acid" as used herein refers to a deoxyribonucleotide (DNA) or ribonucleotide (RNA) in either single- or double-stranded form. The term encompasses nucleic acids containing known analogues of natural nucleotides. The term nucleic acid is used interchangeably with gene, DNA, RNA, cDNA, mRNA, oligonucleotide primer, probe and amplification product. The term also encompasses DNA backbone analogues, such as phosphodiester, phosphorothioate, phosphorodithioate, methylphosphonate, phosphoramidate, alkyl phosphotriester, sulfamate, 3'-thioacetal, methylene (methylimino), 3'-N-carbamate, morpholino carbamate, and peptide nucleic acids (PNAs).

The terms "sample" or "sample of nucleic acids" as used herein refer to a sample comprising a DNA or RNA, or nucleic acid representative of DNA or RNA isolated from a natural source. A "sample of nucleic acids" is in a form suitable for hybridization (e.g., as a soluble aqueous solution) to another nucleic acid (e.g., immobilized probes). The sample nucleic acid may be isolated, cloned, or extracted from particular cells or tissues. The cell or tissue sample from which the nucleic acid sample is prepared is typically taken from a patient having or suspected of having UC or a related disease or condition. Methods of isolating cell and tissue samples are well known to those of skill in the art and include, but are not limited to, aspirations, tissue sections, needle biopsies, and the like. Frequently the sample will be a "clinical sample" which is a sample derived from a patient, including sections of tissues such as frozen sections or paraffin sections taken for histological purposes. The sample can also be derived from supernatants (of cells) or the cells themselves taken from patients or from cell cultures, cells from tissue culture and other media in which it may be desirable to detect the response to drug candidates. In some cases, the nucleic acids may be amplified using standard techniques such as PCR, prior to the hybridization. The probe an be produced from and collectively can be representative of a source of nucleic acids from one or more particular (pre-selected) portions of, e.g., a collection of polymerase chain reaction (PCR) amplification products, substantially an entire chromosome or a chromosome fragment, or substantially an entire genome, e.g., as a collection of clones, e.g., BACs, PACs, YACs, and the like (see below).

"Nucleic acids" are polymers of nucleotides, wherein a nucleotide comprises a base linked to a sugar which sugars are in turn linked one to another by an interceding at least bivalent molecule, such as phosphoric acid. In naturally occurring nucleic acids, the sugar is either 2'-deoxyribose (DNA) or ribose (RNA). Unnatural poly- or oliogonucleotides contain modified bases, sugars, or linking molecules, but are generally understood to mimic the complementary nature of the naturally occurring nucleic acids after which they are designed. An example of an unnatural oligonucleotide is an antisense molecule composition that has a phosphorothiorate backbone. An "oligonucleotide" generally refers to a nucleic acid molecule having less than 30 nucleotides.

The term "profile" means a pattern and relates to the magnitude and direction of change of a number of features. The profile may be interpreted stringently, i.e., where the variation in the magnitude and/or number of features within the profile displaying the characteristic is substantially similar to a reference profile or it may be interpreted less stringently, for example, by requiring a trend rather than an absolute match of all or a subset of feature characteristics.

The terms "protein," "polypeptide," and "peptide" include "analogs," or "conservative variants" and "mimetics" or "peptidomimetics" with structures and activity that substantially correspond to the polypeptide from which the variant was derived, as discussed in detail above.

A "polypeptide" is a polymer of amino acid residues joined by peptide bonds, and a peptide generally refers to amino acid polymers of 12 or less residues. Peptide bonds can be produced naturally as directed by the nucleic acid template or synthetically by methods well known in the art.

A "protein" is a macromolecule comprising one or more polypeptide chains. A protein may further comprise substituent groups attached to the side groups of the amino acids not involved in formation of the peptide bonds. Typically, proteins formed by eukaryotic cell expression also contain carbohydrates. Proteins are defined herein in terms of their amino acid sequence or backbone and substituents are not specified, whether known or not.

The term "receptor" denotes a molecule having the ability to affect biological activity, in e.g., a cell, as a result of interaction with a specific ligand or binding partner. Cell membrane bound receptors are characterized by an extracellular ligand-binding domain, one or more membrane spanning or transmembrane domains, and an intracellular effector domain that is typically involved in signal transduction. Ligand binding to cell membrane receptors causes changes in the extracellular domain that are communicated across the cell membrane, direct or indirect interaction with one or more intracellular proteins, and alters cellular properties, such as enzyme activity, cell shape, or gene expression profile. Receptors may also be untethered to the cell surface and may be cytosolic, nuclear, or released from the cell altogether. Non-cell associated receptors are termed soluble receptors or ligands.

following references are relevant: Ausubel, et al., ed., Current Protocols in Molecular Biology, John Wiley & Sons, Inc., NY (1987-2001); Sambrook, et al., Molecular Cloning: A Laboratory Manual, 2nd Edition, Cold Spring Harbor, NY (1989); Harlow and Lane, antibodies, a Laboratory Manual, Cold Spring Harbor, NY (1989); Colligan, et al., eds., Current Protocols in Immunology, John Wiley & Sons, Inc., NY (1994-2001); Colligan et al., Current Protocols in Protein Science, John Wiley & Sons, NY (1997-2001).

### Gene Panel Identification and Validation

The present disclosure provides novel methods for screening for compositions which modulate the symptoms of UC, particularly the mucosal layer of the rectum and all or part of the colon. By "UC" or grammatical equivalents as used herein, is meant a disease state or condition which is marked by diarrhea, rectal bleeding, tenesmus, passage of mucus, and crampy abdominal pain.

In one aspect, the expression levels of genes are determined in different patient samples for which diagnosis information is desired, to provide expression profiles. An expression profile of a particular sample is essentially a "fingerprint" of the state of the sample; while two states may have any particular gene similarly expressed, the evaluation of a number of genes simultaneously allows the generation of a gene expression profile that is unique to the state of the patient sample. That is, normal tissue may be distinguished from lesion tissue and tissue from a treated patient may be distinguished from an untreated patient. By comparing expression profiles of tissue in different disease states that are known, information regarding which genes are important (including both up- and down-regulation of genes) in each of these states is obtained.

The identification of sequences (genes) that are differentially expressed in disease tissue allows the use of this information in a number of ways. For example, the evaluation of a particular treatment regime may be evaluated.

This may be done by making biochips comprising sets of the important disease genes, which can then be used in these screens. These methods can also be performed on the protein basis; that is, protein expression levels of the UC-related gene product proteins can be evaluated for diagnostic purposes or to screen candidate agents. In addition, the nucleic acid sequences comprising the UC-related gene profile can be used to measure whether a patient is likely to respond to a therapeutic prior to treatment.

UC-related gene sequences can include both nucleic acid and amino acid sequences. In a preferred embodiment, the UC-related gene sequences are recombinant nucleic acids. By the term "recombinant nucleic acid" herein is meant nucleic acid, originally formed in vitro, in general, by the manipulation of nucleic acid by polymerases and endonucleases, in a form not normally found in nature. Thus, an isolated nucleic acid, in a linear form, or an expression vector formed in vitro by ligating DNA molecules that are not normally joined, are both considered recombinant for the purposes of this invention. It is understood that once a recombinant nucleic acid is made and reintroduced into a host cell or organism, it will replicate non-recombinantly, i.e., using the in vivo cellular machinery of the host cell rather than in vitro manipulations; however, such nucleic acids, once produced recombinantly, although subsequently replicated non-recombinantly, are still considered recombinant for the purposes of the invention.

### Method of Practicing the Invention

The invention provides in silico, array-based methods relying on the relative amount of a binding molecule (e.g., nucleic acid sequence) in two or more samples. Also provided are computer- implemented methods for determining the relative amount of a binding molecule (e.g., nucleic acid sequence) in two or more samples and using the determined relative binding amount to predict responsiveness to a particular therapy, and monitor and enhance therapeutic treatment.

In practicing the methods of the disclosure, two or more samples of labeled biological molecules (e.g., nucleic acid) are applied to two or more arrays, where the arrays have substantially the same complement of immobilized binding molecule (e.g., immobilized nucleic acid capable of hybridizing to labeled sample nucleic acid). The two or more arrays are typically multiple copies of the same array. However, because each "spot," "clone" or "feature" on the array has similar biological molecules (e.g., nucleic acids of the same sequence) and the biological molecules (e.g., nucleic acid) in each spot is known, as is typical of nucleic acid and other arrays, it is not necessary that the multiple arrays used in the invention be identical in configuration it is only necessary that the position of each feature on the substrate be known, that is, have an address. Thus, in one aspect, multiple biological molecules (e.g., nucleic acid) in samples are comparatively bound to the array (e.g., hybridized simultaneously) and the information gathered is coded so that the results are based on the inherent properties of the feature (e.g., the nucleic acid sequence) and not it's position on the substrate.

### Amplification of Nucleic Acids

Amplification using oligonucleotide primers can be used to generate nucleic acids used in the compositions and methods of the invention, to detect or measure levels of test or control samples hybridized to an array, and the like. The skilled artisan can select and design suitable oligonucleotide amplification primers. Amplification methods are also well known in the art, and include, e.g., polymerase chain reaction, PCR (PCR PROTOCOLS, A GUIDE TO METHODS AND APPLICATIONS, ed. Innis, Academic Press, N.Y. (1990) and PCR STRATEGIES (1995), ed. Innis, Academic Press, Inc., N.Y., ligase chain reaction (LCR) (see, e.g., Wu (1989) Genomics 4:560; Landegren (1988) Science 241:1077; Barringer (1990) Gene 89:117); transcription amplification (see, e.g., Kwoh (1989) Proc. Natl. Acad. Sci. USA 86:1173); and, self-sustained sequence replication (see, e.g., Guatelli (1990) Proc. Natl. Acad. Sci. USA 87:1874); Q Beta replicase amplification (see, e.g., Smith (1997) J. Clin. Microbiol. 35:1477-1491), automated Q-beta replicase amplification assay (see, e.g., Burg (1996) Mol. Cell. Probes 10:257-271) and other RNA polymerase mediated techniques (e.g., NASBA, Cangene, Mississauga, Ontario); see also Berger (1987) Methods Enzymol. 152:307-316; Sambrook; Ausubel; U.S. Pat. Nos. 4,683,195 and 4,683,202; Sooknanan (1995) Biotechnology 13:563-564.

### Hybridizing Nucleic Acids

In practicing the methods of the disclosure, test and control samples of nucleic acid are hybridized to immobilized probe nucleic acid, e.g., on arrays. In alternative aspects, the hybridization and/or wash conditions are carried out under moderate conditions, stringent conditions and very stringent conditions. An extensive guide to the hybridization of nucleic acids is found in, e.g., Sambrook Ausubel, Tijssen. Generally, highly stringent hybridization and wash conditions are selected to be about 5°C lower than the thermal melting point (Tm) for the specific sequence at a defined ionic strength and pH. The Tm is the temperature (under defined ionic strength and pH) at which 50% of the target sequence hybridizes to a perfectly matched probe. Very stringent conditions are selected to be equal to the Tm for a particular probe. An example of stringent hybridization conditions for hybridization of complementary nucleic acids which have more than 100 complementary residues on an array or a filter in a Southern or northern blot is 42°C using standard hybridization solutions (see, e.g., Sambrook), with the hybridization being carried out overnight. An example of highly stringent wash conditions is 0.15 M NaCl at 72°C for about 15 minutes. An example of stringent wash conditions is a 0.2xSSC wash at 65°C for 15 minutes (see, e.g., Sambrook). Often, a high stringency wash is preceded by a medium or low stringency wash to remove background probe signal. An example medium stringency wash for a duplex of, e.g., more than 100 nucleotides, is 1×SSC at 45°C for 15 minutes. An example of a low stringency wash for a duplex of, e.g., more than 100 nucleotides, is 4x to 6xSSC at 40° C for 15 minutes.

In alternative aspects of the compositions and methods e.g., in practicing comparative nucleic acid hybridization, such as comparative genomic hybridization (CGH) with arrays, the fluorescent dyes Cy3® and Cy5® are used to differentially label nucleic acid fragments from two samples, e.g., the array-immobilized nucleic acid versus the sample nucleic acid, or, nucleic acid generated from a control versus a test cell or tissue. Many commercial instruments are designed to accommodate the detection of these two dyes. To increase the stability of Cy5®, or fluors or other oxidation-sensitive compounds, antioxidants and free radical scavengers can be used in hybridization mixes, the hybridization and/or the wash solutions. Thus, Cy5® signals are dramatically increased and longer hybridization times are possible. See WO 0194630 A2 and U.S. Patent Application No. 20020006622.

To further increase the hybridization sensitivity, hybridization can be carried out in a controlled, unsaturated humidity environment; thus, hybridization efficiency is significantly improved if the humidity is not saturated. See WO 0194630 A2 and U.S. Patent Application No. 20020006622. The hybridization efficiency can be improved if the humidity is dynamically controlled, i.e., if the humidity changes during hybridization. Mass transfer will be facilitated in a dynamically balanced humidity environment. The humidity in the hybridization environment can be adjusted stepwise or continuously. Array devices comprising housings and controls that allow the operator to control the humidity during pre-hybridization, hybridization, wash and/or detection stages can be used. The device can have detection, control and memory components to allow pre-programming of the humidity and temperature controls (which are constant and precise or which flucturate), and other parameters during the entire procedural cycle, including pre-hybridization, hybridization, wash and detection steps. See WO 0194630 A2 and U.S. Patent Application No. 20020006622.

The methods can comprise hybridization conditions comprising osmotic fluctuation. Hybridization efficiency (i.e., time to equilibrium) can also be enhanced by a hybridization environment that comprises changing hyper-/hypo-tonicity, e.g., a solute gradient. A solute gradient is created in the device. For example, a low salt hybridization solution is placed on one side of the array hybridization chamber and a higher salt buffer is placed on the other side to generate a solute gradient in the chamber. See WO 0194630 A2 and U.S. Patent Application No. 20020006622.

### Blocking the Ability of Repetitive Nucleic Acid Sequences to Hybridize

The methods can comprise a step of blocking the ability of repetitive nucleic acid sequences to hybridize (i.e., blocking "hybridization capacity") in the immobilized nucleic acid segments. The hybridization capacity of repetitive nucleic acid sequences in the sample nucleic acid sequences can be blocked by mixing sample nucleic acid sequences with unlabeled or alternatively labeled repetitive nucleic acid sequences. Sample nucleic acid sequences can be mixed with repetitive nucleic acid sequences before the step of contacting with the array-immobilized nucleic acid segments. Blocking sequences are for example, Cot-1 DNA, salmon sperm DNA, or specifc repetitive genomic sequences. The repetitive nucleic acid sequences can be unlabeled. A number of methods for removing and/or disabling the hybridization capacity of repetitive sequences using, e.g., Cot-1 are known; see, e.g., Craig (1997) Hum. Genet. 100:472-476; WO 93/18186. Repetitive DNA sequences can be removed from library probes by means of magnetic purification and affinity PCR, see, e.g., Rauch (2000) J. Biochem. Biophys. Methods 44:59-72.

Arrays are generically a plurality of target elements immobilized onto the surface of the plate as defined "spots" or "clusters," or "features," with each target element comprising one or more biological molecules (e.g., nucleic acids or polypeptides) immobilized to a solid surface for specific binding (e.g., hybridization) to a molecule in a sample. The immobilized nucleic acids can contain sequences from specific messages (e.g., as cDNA libraries) or genes (e.g., genomic libraries), including a human genome. Other target elements can contain reference sequences and the like. The biological molecules of the arrays may be arranged on the solid surface at different sizes and different densities. The densities of the biological molecules in a cluster and the number of clusters on the array will depend upon a number of factors, such as the nature of the label, the solid support, the degree of hydrophobicity of the substrate surface, and the like. Each feature may comprise substantially the same biological molecule (e.g., nucleic acid), or, a mixture of biological molecules (e.g., nucleic acids of different lengths and/or sequences). Thus, for example, a feature may contain more than one copy of a cloned piece of DNA, and each copy may be broken into fragments of different lengths.

Array substrate surfaces onto which biological molecules (e.g., nucleic acids) are immobilized can include nitrocellulose, glass, quartz, fused silica, plastics and the like, as discussed further, below. The compositions and methods can incorporate in whole or in part designs of arrays, and associated components and methods, as described, e.g., in U.S. Pat. Nos. 6,344,316; 6,197,503; 6,174,684; 6,159,685; 6,156,501; 6,093,370; 6,087,112; 6,087, 103; 6,087,102; 6,083,697; 6, 080,585; 6,054,270; 6,048,695; 6,045,996; 6,022,963; 6,013,440; 5,959,098; 5,856,174; 5,843,655; 5,837,832; 5,770,456; 5,723,320; 5,700,637; 5,695, 940; 5,556,752; 5,143,854; see also, e.g., WO 99/51773; WO 99/09217; WO 97/46313; WO 96/17958; WO 89/10977; see also, e.g., Johnston (1998) Curr. Biol. 8:R171-174; Schummer (1997) Biotechniques 23:1087-1092; Kern (1997) Biotechniques 23:120-124; Solinas- Toldo (1997) Genes, Chromosomes & Cancer 20:399-407; Bowtell (1999) Nature Genetics Supp. 21:25-32; Epstein (2000) Current Opinion in Biotech. 11:36-41; Mendoza (1999 Biotechniques 27: 778-788; Lueking (1999) Anal. Biochem. 270:103-111; Davies (1999) Biotechniques 27:1258-1261.

### Substrate Surfaces

Substrate surfaces that can be used in the compositions and methods include, for example, glass (see, e.g., U.S. Pat. No. 5,843,767), ceramics, and quartz. The arrays can have substrate surfaces of a rigid, semi-rigid or flexible material. The substrate surface can be flat or planar, be shaped as wells, raised regions, etched trenches, pores, beads, filaments, or the like. Substrate surfaces can also comprise various materials such as nitrocellulose, paper, crystalline substrates (e.g., gallium arsenide), metals, metalloids, polacryloylmorpholide, various plastics and plastic copolymers, Nylon®, Teflon®, polyethylene, polypropylene, latex, polymethacrylate, poly (ethylene terephthalate), rayon, nylon, poly(vinyl butyrate), and cellulose acetate. The substrates may be coated and the substate and the coating may be functionalized to, e.g., enable conjugation to an amine.

### Arrays Comprising Calibration Sequences

The disclosure comtemplates the use of arrays comprising immobilized calibration sequences for normalizing the results of array-based hybridization reactions, and methods for using these calibration sequences, e.g., to determine the copy number of a calibration sequence to "normalize" or "calibrate" ratio profiles. The calibration sequences can be substantially the same as a unique sequence in an immobilized nucleic acid sequence on an array. For example, a "marker" sequence from each "spot" or "biosite" on an array (which is present only on that spot, making it a "marker" for that spot) is represented by a corresponding sequence on one or more "control" or "calibration" spot(s).

The "control spots" or "calibration spots" are used for "normalization" to provide information that is reliable and repeatable. Control spots can provide a consistent result independent of the labeled sample hybridized to the array (or a labeled binding molecule from a sample). The control spots can be used to generate a "normalization" or "calibration" curve to offset possible intensity errors between the two arrays (or more) used in the in silico, array-based methods of the disclosure.

One method of generating a control on the array would be to use an equimolar mixture of all the biological molecules (e.g., nucleic acid sequences) spotted on the array and generating a single spot. This single spot would have equal amounts of the biological molecules (e.g., nucleic acid sequences) from all the other spots on the array. Multiple control spots can be generated by varying the concentration of the equimolar mixture.

### Samples and Specimens

The sample nucleic acid may be isolated, cloned, or extracted from particular cells, tissues, or other specimens. The cell or tissue sample from which the nucleic acid sample is prepared is typically taken from a patient having or suspected of having UC or a related condition. Methods of isolating cell and tissue samples are well known to those of skill in the art and include, but are not limited to, aspirations, tissue sections, needle biopsies, and the like. Frequently, the sample will be a "clinical sample" which is a sample derived from a patient, including whole blood, or sections of tissues, such as frozen sections or paraffin sections taken for histological purposes. The sample can also be derived from supernatants (of cells) or the cells themselves taken from patients or from cell cultures, cells from tissue culture and other media in which it may be desirable to detect the response to drug candidates. In some cases, the nucleic acids may be amplified using standard techniques such as PCR, prior to the hybridization.

In one embodiment, the present invention is a pre-treatment method of predicting disease regression or resolution. The method includes (1) taking a colon biopsy or other specimen from an individual diagnosed with UC or a related disease or disorder, (2) measuring the expression levels of the profile genes of the panel, (3) comparing the pre-treatment expression level of the genes with a pre-treatment reference profile from treatment responders, and (4) predicting treatment response by monitoring the expression levels of the gene panel.

### Methods of Assessing Biomarker Utililty

The prognostic utility of the present biomarker gene panel for assessing a patient's response to treatment or prognosis of disease can be validated by using other means for assessing a patient's state of disease. For example, gross measurement of disease may be assessed and recorded by certain imaging methods, such as but not limited to: imaging by photographic, radiometric, or magnetic resonance technology. General indices of health or disease futher include serum or blood composition (protein, liver enzymes, pH, electrolytes, red cell volume, hematocrit, hemoglobin, or specific protein). However, in some diseases, the etiology is still poorly understood. UC is an example of one such disease.

### Patient Assessment and Monitoring

Some of the genes in the panel belong to classes of genes that have been reported to be aberrantly expressed in UC patients previously, such as transcription factors, replication proteins, and oxidases, the expression patterns of the genes over the course of treatment have not been studied in the treatment of UC, and none has been identified as having predictive value. The panel of gene expression biomarkers disclosed herein permits the generation of methods for rapid and reliable prediction, diagnostic tools that predict the clinical outcome of a UC trial, or prognostic tools for tracking the efficacy of UC therapy. Prognostic methods based on detecting these genes in a sample are provided. These compositions may be used, for example, in connection with the diagnosis, prevention and treatment of a range of immune-mediated inflammatory diseases.

### Therapeutic agents

### Antagonists

As used herein, the term "antagonists" refer to substances which inhibit or neutralize the biologic activity of the gene product of the UC-related gene panel of the disclosure. Such antagonists accomplish this effect in a variety of ways. One class of antagonists will bind to the gene product protein with sufficient affinity and specificity to neutralize the biologic effects of the protein. Included in this class of molecules are antibodies and antibody fragments (such as, for example, F(ab) or F(ab')₂ molecules). Another class of antagonists comprises fragments of the gene product protein, muteins or small organic molecules, i.e., peptidomimetics, that will bind to the cognate binding partners or ligands of the gene product, thereby inhibiting the biologic activity of the specific interaction of the gene product with its cognate ligand or receptor. The UC-related gene antagonist may be of any of these classes as long as it is a substance that inhibits at least one biological activity of the gene product.

Antagonists include antibodies directed to one or more regions of the gene product protein or fragments thereof, antibodies directed to the cognate ligand or receptor, and partial peptides of the gene product or its cognate ligand which inhibit at least one biological activity of the gene product. Another class of antagonists includes siRNAs, shRNAs, antisense molecules and DNAzymes targeting the gene sequence as known in the art are disclosed herein.

Suitable antibodies include those that compete for binding to UC-related gene products with monoclonal antibodies that block UC-related gene product activation or prevent UC-related gene product binding to its cognate ligand, or prevent UC-related gene product signalling.

A therapeutic targeting the inducer of the UC-related gene product may provide better chances of success. Gene expression can be modulated in several different ways including by the use of siRNAs, shRNAs, antisense molecules and DNAzymes. Synthetic siRNAs, shRNAs, and DNAzymes can be designed to specifically target one or more genes and they can easily be delivered to cells in vitro or in vivo.

The present disclosure encompasses antisense nucleic acid molecules, i.e., molecules that are complementary to a sense nucleic acid encoding a UC-related gene product polypeptide, e.g., complementary to the coding strand of a double-stranded cDNA molecule or complementary to an mRNA sequence. Accordingly, an antisense nucleic acid can hydrogen bond to a sense nucleic acid. The antisense nucleic acid can be complementary to an entire coding strand, or to only a portion thereof, e.g., all or part of the protein coding region (or open reading frame). An antisense nucleic acid molecule can be antisense to all or part of a non-coding region of the coding strand of a nucleotide sequence encoding a UC-related gene product polypeptide. The non-coding regions ("5' and 3' untranslated regions") are the 5' and 3' sequences that flank the coding region and are not translated into amino acids.

The disclosure also provides chimeric or fusion proteins. As used herein, a "chimeric protein" or "fusion protein" comprises all or part (preferably biologically active) of a UC-related gene product polypeptide operably linked to a heterologous polypeptide (i.e., a polypeptide other than the same UC-related gene product polypeptide). Within the fusion protein, the term "operably linked" is intended to indicate that the UC-related gene product polypeptide and the heterologous polypeptide are fused in-frame to each other. The heterologous polypeptide can be fused to the amino-terminus or the carboxyl-terminus of the UC-related gene product polypeptide. In another embodiment, a UC-related gene product polypeptide or a domain or active fragment thereof can be fused with a heterologous protein sequence or fragment thereof to form a chimeric protein, where the polypeptides, domains or fragments are not fused end to end but are interposed within the heterologous protein framework.

In yet another embodiment, the fusion protein is an immunoglobulin fusion protein in which all or part of a UC-related gene product polypeptide is fused to sequences derived from a member of the immunoglobulin protein family. The immunoglobulin fusion proteins of the disclosure can be incorporated into pharmaceutical compositions and administered to a subject to inhibit an interaction between a ligand (soluble or membrane-bound) and a protein on the surface of a cell (receptor), to thereby suppress signal transduction in vivo. The immunoglobulin fusion protein can be used to affect the bioavailability of a cognate ligand of a UC-related gene product polypeptide. Inhibition of ligand/receptor interaction can be useful therapeutically, both for treating proliferative and differentiative disorders and for modulating (e.g., promoting or inhibiting) cell survival. A preferred embodiment of an immunoglobulin chimeric protein is a C_{H}1 domain-deleted immunoglobulin or MIMETIBODY™ construct having an active polypeptide fragment interposed within a modified framework region as taught in copending application PCT WO/04002417. Moreover, the immunoglobulin fusion proteins of the disclosure can be used as immunogens to produce antibodies directed against a UC-related gene product polypeptide in a subject, to purify ligands and in screening assays to identify molecules that inhibit the interaction of receptors with ligands.

### Compositions and Their Uses

In accordance with the disclosure, the neutralizing anti-UC-related gene product antagonists, such as monoclonal antibodies, described herein can be used to inhibit UC-related gene product activity. Additionally, such antagonists can be used to inhibit the pathogenesis of UC and -related inflammatory diseases amenable to such treatment, which may include, but are not limited to, rheumatic diseases. The individual to be treated may be any mammal and is preferably a primate, a companion animal which is a mammal and most preferably a human patient. The amount of antagonist administered will vary according to the purpose it is being used for and the method of administration.

The UC-related gene antagonists may be administered by any number of methods that result in an effect in tissue in which pathological activity is desired to be prevented or halted. Further, the anti-UC-related gene product antagonists need not be present locally to impart an effect on the UC-related gene product activity, therefore, they may be administered wherever access to body compartments or fluids containing UC-related gene product is achieved. In the case of inflamed, malignant, or otherwise compromised tissues, these methods may include direct application of a formulation containing the antagonists. Such methods include intravenous administration of a liquid composition, transdermal administration of a liquid or solid formulation, oral, topical administration, or interstitial or inter-operative administration. Adminstration may be affected by the implantation of a device whose primary function may not be as a drug delivery vehicle.

For antibodies, the preferred dosage is about 0.1 mg/kg to 100 mg/kg of body weight (generally about 10 mg/kg to 20 mg/kg). If the antibody is to act in the brain, a dosage of about 50 mg/kg to 100 mg/kg is usually appropriate. Generally, partially human antibodies and fully human antibodies have a longer half-life within the human body than other antibodies. Accordingly, the use of lower dosages and less frequent administration is often possible. Modifications, such as lipidation, can be used to stabilize antibodies and to enhance uptake and tissue penetration (e.g., into the brain). A method for lipidation of antibodies is described by Cruikshank et al. ((1997) J. Acquired Immune Deficiency Syndromes and Human Retrovirology 14:193).

The UC-related gene product antagonist nucleic acid molecules can be inserted into vectors and used as gene therapy vectors. Gene therapy vectors can be delivered to a subject by, for example, intravenous injection, local administration (U.S. Pat. No. 5,328,470), or by stereotactic injection (see, e.g., Chen et al. (1994) Proc. Natl. Acad. Sci. USA 91:3054- 3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

The pharmaceutical compositions can be included in a container, pack, or dispenser together with instructions for administration.

### Pharmacogenomics

Agents, or modulators that have a stimulatory or inhibitory effect on activity or expression of a UC-related gene product polypeptide as identified by a screening assay described herein, can be administered to individuals to treat (prophylactically or therapeutically) disorders associated with aberrant activity of the polypeptide. In conjunction with such treatment, the pharmacogenomics (i.e., the study of the relationship between an individual's genotype and that individual's response to a foreign compound or drug) of the individual may be considered. Differences in metabolism of therapeutics can lead to severe toxicity or therapeutic failure by altering the relation between dose and blood concentration of the pharmacologically active drug. Thus, the pharmacogenomics of the individual permits the selection of effective agents (e.g., drugs) for prophylactic or therapeutic treatments based on a consideration of the individual's genotype. Such pharmacogenomics can further be used to determine appropriate dosages and therapeutic regimens. Accordingly, the activity of a UC-related gene product polypeptide, expression of a UC-related gene product nucleic acid, or mutation content of a UC-related gene product gene in an individual can be determined to thereby select an appropriate agent(s) for therapeutic or prophylactic treatment of the individual.

Pharmacogenomics deals with clinically significant hereditary variations in the response to drugs due to altered drug disposition and abnormal action in affected persons. See, e.g., Linder (1997) Clin. Chem. 43(2): 254-266. In general, two types of pharmacogenetic conditions can be differentiated. Genetic conditions transmitted as a single factor altering the way drugs act on the body are referred to as "altered drug action." Genetic conditions transmitted as single factors altering the way the body acts on drugs are referred to as "altered drug metabolism." These pharmacogenetic conditions can occur either as rare defects or as polymorphisms. For example, glucose-6-phosphate dehydrogenase (G6PD) deficiency is a common inherited enzymopathy in which the main clinical complication is hemolysis after ingestion of oxidant drugs (anti-malarials, sulfonamides, analgesics, nitrofurans) and consumption of fava beans.

As an illustrative embodiment, the activity of drug metabolizing enzymes is a major determinant of both the intensity and duration of drug action. The discovery of genetic polymorphisms of drug metabolizing enzymes (e.g., N-acetyltransferase 2 (NAT 2) and cytochrome P450 enzymes CYP2D6 and CYP2C19) has provided an explanation as to why some patients do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutations have been identified in PM, which all lead to the absence of functional CYP2D6. Poor metabolizers of CYP2D6 and CYP2C19 quite frequently experience exaggerated drug response and side effects when they receive standard doses. If a metabolite is the active therapeutic moiety, a PM will show no therapeutic response, as demonstrated for the analgesic effect of codeine mediated by its CYP2D6-formed metabolite morphine. The other extreme are the so called ultra-rapid metabolizers who do not respond to standard doses. Recently, the molecular basis of ultra-rapid metabolism has been identified to be due to CYP2D6 gene amplification.

Thus, the activity of a UC-related gene product polypeptide, expression of a nucleic acid encoding the polypeptide, or mutation content of a gene encoding the polypeptide in an individual can be determined to thereby select appropriate agent(s) for therapeutic or prophylactic treatment of the individual. In addition, pharmacogenetic studies can be used to apply genotyping of polymorphic alleles encoding drug-metabolizing enzymes to the identification of an individual's drug responsiveness phenotype. This knowledge, when applied to dosing or drug selection, can avoid adverse reactions or therapeutic failure and thus enhance therapeutic or prophylactic efficiency when treating a subject with a modulator of activity or expression of the polypeptide, such as a modulator identified by one of the exemplary screening assays described herein.

### Methods of Treatment

The present disclosure provides for both prophylactic and therapeutic methods of treating a subject at risk of (or susceptible to) a disorder or having a disorder associated with aberrant expression or activity of a UC-related gene product polypeptide and/or in which the UC-related gene product polypeptide is involved.

The present disclosure provides a method for modulating or treating at least one UC-related gene product related disease or condition, in a cell, tissue, organ, animal, or patient, as known in the art or as described herein, using at least one UC-related gene product antagonist.

Compositions of UC-related gene product antagonist may find therapeutic use in the treatment of UC or related conditions, such as Crohn's disease or other gastrointestinal disorders.

The present disclosure also provides a method for modulating or treating at least one gastrointestinal, immune related disease, in a cell, tissue, organ, animal, or patient including, but not limited to, at least one of gastric ulcer, inflammatory bowel disease, ulcerative colitis, Crohn's pathology, and the like. See, e.g., the Merck Manual, 12th-17th Editions, Merck & Company, Rahway, NJ (1972, 1977, 1982, 1987, 1992, 1999), Pharmacotherapy Handbook, Wells et al., eds., Second Edition, Appleton and Lange, Stamford, Conn. (1998, 2000).

Disorders characterized by aberrant expression or activity of the UC-related gene product polypeptides are further described elsewhere in this disclosure.

### 1. Prophylactic Methods

In one aspect, the disclosure provides a method for at least substantially preventing in a subject, a disease or condition associated with an aberrant expression or activity of a UC-related gene product polypeptide, by administering to the subject an agent that modulates expression or at least one activity of the polypeptide. Subjects at risk for a disease that is caused or contributed to by aberrant expression or activity of a UC-related gene product can be identified by, for example, any or a combination of diagnostic or prognostic assays as described herein. Administration of a prophylactic agent can occur prior to the manifestation of symptoms characteristic of the aberrancy, such that a disease or disorder is prevented or, alternatively, delayed in its progression. Depending on the type of aberrancy, for example, an agonist or antagonist agent can be used for treating the subject. The appropriate agent can be determined based on screening assays described herein.

### 2. Therapeutic Methods

Another aspect of the disclosure pertains to methods of modulating expression or activity of UC-related gene or gene product for therapeutic purposes. The modulatory method of the disclosure involves contacting a cell with an agent that modulates one or more of the activities of the polypeptide. An agent that modulates activity can be an agent as described herein, such as a nucleic acid or a protein, a naturally-occurring cognate ligand of the polypeptide, a peptide, a peptidomimetic, or other small molecule. In one embodiment, the agent stimulates one or more of the biological activities of the polypeptide. In another embodiment, the agent inhibits one or more of the biological activities of the UC-related gene or gene product polypeptide. Examples of such inhibitory agents include antisense nucleic acid molecules and antibodies and other methods described herein. These modulatory methods can be performed in vitro (e.g., by culturing the cell with the agent) or, alternatively, in vivo (e.g., by administering the agent to a subject). As such, the present disclosure provides methods of treating an individual afflicted with a disease or disorder characterized by aberrant expression or activity of a UC-related gene product polypeptide. In one embodiment, the method involves administering an agent (e.g., an agent identified by a screening assay described herein), or combination of agents that modulate (e.g., up-regulates or down-regulates) expression or activity. Inhibition of activity is desirable in situations in which activity or expression is abnormally high or up-regulated and/or in which decreased activity is likely to have a beneficial effect.

### EXAMPLE 1: Sample Collection and Analysis

### Patients

Twenty-three biopsies obtained at Week 0 were analyzed from a subgroup of 22 patients who received either infliximab (IFX) 5 or 10 mg/kg (11 biopsies from 10 nonresponders and 12 biopsies from 12 responders; two of the nonresponder biopsies were obtained within two weeks from the same subject). Messenger RNA was isolated from pre-infliximab biopsies, labeled and hybridized to Affymetrix HGU133Plus_2.0 Array. The predictive response signature was verified by an independent data set. The trial design of ACT1 (ClinTrials.gov Identifier NCT00036439), including patient eligibility criteria, randomization, and trial procedures has been previously described in detail.

Biopsies were collected at protocol-specified time points from a subset of ACT1 randomized patients. All biopsies were collected in accordance with Institutional Review Board regulations. The institutional review board or ethics committee at each site approved the protocol and all patients provided informed consent.

Response was determined at week 8. Response to infliximab was defined as complete mucosal healing (i.e., Mayo endoscopic subscore of 0 or 1) and a grade of 0 or 1 on the histological score for UC. Patients who did not achieve mucosal healing were considered nonresponders, although some patients showed histologic improvement. The baseline characteristics of this cohort are presented in Table 1 below.

### Intestinal biopsy samples and RNA preparation

Biopsies were collected 15 to 20 centimeters distal from the anal verge during endoscopies conducted at Week 0. Biopsies were quick frozen in liquid nitrogen, and stored at -80°C until processing. Total RNA was isolated with RNeasy mini-kit according to the manufacturer's instructions (Qiagen Inc., Valencia, CA). RNA quality and quantity were analyzed with a 2100 Bioanalyzer (Agilent Technologies Inc., Palo Alto, CA).

An independent validation cohort of biopsies from 24 UC patients treated with infliximab was obtained from University Hospital Gasthuisberg, Leuven Belgium. Biopsies were obtained within one week prior to the intravenous infusion of 5 mg/kg infliximab. The biopsies were frozen at -80°C prior to processing for mRNA expression. The response to infliximab was determined at 4-6 weeks post-infusion, with response defined as above. The Leuven cohort specimens were processed for mRNA isolation and hybridization using the same methods as were used for the ACT1 specimens.

### Microarray hybridization

Microarray hybridization was performed on GeneChip Human Genome U133 Plus 2.0 arrays according to the manufacturer's protocol (Affymetrix, Santa Clara, CA). This chip allows expression level analysis of more than 47,000 transcripts and variants, including 38,500 well-characterized human genes. The chips were scanned with a GeneChip Scanner 3000, and fluorescence intensity for each feature of the array was obtained with GeneChip Operating Software version 1.4 (Affymetrix, Santa Clara, CA).

### Microarray data analysis

Data quality was assessed by hybridization intensity distribution and Pearson's correlation with Partek Genomic Suite 6.3 (Partek Inc., St. Charles, MO). Pearson's correlation coefficients ranged from 0.80 to 1.0. The intensity of probe sets was normalized across all samples using GeneSpringGX 7.3 (Agilent Technologies, Palo Alto, CA).

Significant differences between infliximab nonresponder and responder samples were identified using analysis of variance (ANOVA) on log-2 transformed normalized intensities. A 5% false discovery rate (FDR) was applied for multiple testing corrections. Transcripts with more than 2-fold differential expression were selected for the comparison to be analyzed. To exclude probe sets that passed ANOVA and fold change filtering but were undetected in both conditions of a paired comparison, only those samples designated as "present" (detected) or "marginal" (limited detection) at least once among samples representing the condition were selected.

### Class prediction analysis

Classification of infliximab responsiveness for each patient sample was generated with the 'K-Nearest Neighbours" algorithm using GeneSpring GX 7.3. A classifier containing transcripts showing significant differential expression between nonresponder (n=11 samples) and responder (n=12 samples) before infliximab treatment was evaluated by leave-one-out cross-validation. A p-value was calculated to measure the probability of a test sample being classified by chance. Fisher's Exact Test was used to select the top predictive transcripts.

### Unsupervised clustering analysis

Hierarchical clustering analysis was applied to data obtained from the microarray data analysis. Clustering was run using Pearson correlation between the expression profiles of two genes or patients to calculate the similarity matrix in GeneSpringGX 7.3. Results were visualized as a 2-dimensional heat map with two dendrograms (not shown), one indicating the similarity between patients and the other indicating the similarity between genes.

### Functional annotation

Gene-annotation enrichment analysis was conducted using National Institutes of Health DAVID online (http://david.abcc.ncifcrf.gov/). Statistical significance was determined using Fisher's exact test. Functional categories with a p-value ≤ 0.05 were considered significant.

### Baseline gene signature differentiating Responders from Nonresponders

The expression profile of mucosal biopsies at Week 0 before infliximab treatment was established from 22 patients (11 biopsies from 10 nonresponders and 12 biopsies from 12 responders; two of the nonresponder biopsies were obtained within two weeks from the same subject) based upon the response to infliximab at Week 8. A total of 109 probe sets, 102 up-regulated and 7 down-regulated, passed an FDR of 5% and a two-fold differential cut-off representing 90 genes.

When classified into biological processes, there was a predominance of innate immune processes. The five most predominant innate immune processes were defense response, immune response, signal transduction, response to other organisms, and response to pests, pathogens or parasites (Figure 1). Ten probe set members were cytokines/chemokines or cytokine/chemokine receptors. Included in the probe set was CXCL8/interleukin (IL)-8, a chemotactic chemokine for neutrophil polymorphonuclear leucocytes (PMN). The receptors for CXCL8/IL-8, CXCR1/IL-8RA and CXCR2/IL-8RB were also present. Also present was CXCL11/I-TAC, a chemotactic factor that activates T cells and natural killer cells. Finally, IL-1β, IL-1RN, and IL-11, were all upregulated more than four-fold when comparing nonresponders to responders.

### Class prediction analysis of Responders and NonResponders

Fisher's Exact Test was used to select the top predictive probe sets distinguishing nonresponders from responders within the 109 probe sets shown to be differentially expressed at Week 0. A subset of 20 probe sets classified nonresponders and responders at Week 0 (Table 2) with an overall accuracy of 95.4% (21/22), sensitivity of 91.7% (11/12 responders) and specificity of 100% (10/10 nonresponders) (Table 2). Genes involved in immune responses (IL-1β, TLR2, TREM1 and LILRA1), signal transduction (PDE4B, PBEF1 and FCN1) or G-protein-coupled receptor protein signaling pathways (GPR109B, C5AR1 [C5R1] and FPRL1) were represented. Eight of these 20 genes were expressed by PMNs, which are present in large numbers in the colonic mucosa from patients with active UC. Hierarchical clustering of the 20 probe set classifier showed a clear separation among nonresponders and responders (not shown). The minimal number of transcripts allowing for an equivalent classification was subsequently determined. A classifier containing as few as 5 probe sets selected from the above 20 was able to reach an overall accuracy of 90.9% (20/22), sensitivity of 91.7% (11/12 responders) and specificity of 90.0% (9/10 nonresponders) (Tables 2 and 3). The 5 genes obtained were BCL6, CREB5, C5AR1, FPRL1, and OSM. Both BCL6 and CREB5 had slightly higher predictive strength while the remaining 3 genes had equal predictive strength. Of note, any of the remaining 15 genes could replace C5AR1, FPRL1, and OSM without any loss to the predictive quality of the 5 probe set classifier (data not shown). Hierarchical clustering of the 5 probe set classifier across the 10 nonresponders and 12 responders showed remarkable separation (not shown) with a very contrasted expression profile across all 5 probe sets when comparing nonresponders to responders. The single misclassified responder had an expression profile very similar to nonresponders with the exception of CREB5. Finally, Figure 2 shows a dot plot representation of the 5-gene classifier using the normalized raw intensities where a marked difference is seen for each of the five genes at baseline comparing nonresponders to responders.

### Class prediction validation

The 20 and the 5 probe set classifiers were validated using the Leuven cohort as an independent validation test set composed of 16 nonresponders and 8 responders. Overall accuracies of 75% (18/24), sensitivities of 87.5% (7/8 responders) and specificities of 68.8% (11/16 nonresponders) were obtained for both classifiers. Hierarchical clustering of the 20 probe set classifier among the 16 nonresponders and 8 responders showed that the 4 misclassified nonresponders and the 1 misclassified responder have expression profiles very similar to responders and nonresponders, respectively.

These probe sets all passed a 5% FDR and a two-fold differential expression cutoff. Two predictive response signatures, one a 20 probe set and one of 5 sub-set of the 20, were established and verified using an independent cohort. Four nonresponders were misclassified but had expression patterns resembling that of responders for all 109 genes differentially expressed at Week 0 (data not shown). This suggests that these patients are either slow to manifest a clinical benefit from infliximab treatment or have factors influencing their clinical response not evident through mucosal expression profiling at Week 0. One responder from the ACT1 cohort was misclassified by the 20 and 5 probe set classifiers, while one responder in the independent validation cohort was misclassified.
**Utility of the response signature.** The response signature for infliximab treatment in UC described herein can be assessed and used as described below.
1) Colonoscopic biopsy samples are obtained from lesional sites of patients with active UC (or Crohn's or related diseases and disorders). RNA will then be isolated from the biopsy samples and subjected to real time RT-PCR analysis. One microgram of total RNA in the volume of 50 µl is converted to cDNA in the presence of MultiScribe Reverse Transcriptase (ABI biosystem, Foster City, California). The reaction is carried out by incubating for 10 minutes at 25°C followed by 30 minutes at 48°C. Reverse Transcriptase is inactivated at 95°C for 5 minutes. Twenty-five nanograms of cDNA per reaction are used in real time PCR with ABI 7900 system (Foster City, California). In the presence of AmpliTaq Gold DNA polymerase (ABI biosystem, Foster City, California), the reaction is incubated for 2 minutes at 50°C followed by 10 minutes at 95°C. Then the reaction is run for 40 cycles at 15 seconds, at 95°C and 1 minute, 60°C per cycle using primer/probe sets specific for the genes in the response signature. House keeping genes, such as GAPDH or actin, will be used as internal calibrators. The relative change in gene expression is calculated using the delta-delta Ct method described by Applied Biosystems using values in the non-responder samples as the calibrator or comparator.
2) If a similar gene expression profile meets the parameters of the gene profile signature for a type of therapy, i.e., one or more of the 5 or 20 signature genes in the profiles described above show expression levels predictive of responders in relation to non-responders, the patient is considered a likely treatment responder to the therapy. In which case, the patient will be treated with the therapy.
3) If the gene expression profile does not meet the parameters of the gene profile signature for responder, i.e., lower expression level, then the patient is defined as a likely treatment non-responder. In which case, the patient may not be treated with the therapy. This enables a patient to avoid a type of therapy earlier after being deemed a non-responder. This can allow the patient to receive a different type of therapy.

### Comparison method in relation to reference standard:

Total RNA is to be analyzed on a gene chip array for the expression intensities of the 20-gene panel listed in Table 2 or the 5-gene panel listed in bold in Table 2. The following procedures are exemplary of a method of evaluating members of a gene panel of the invention against a reference standard in order to compare values of the 20- or 5-gene panel members:
1. Total RNA is extracted from a biopsy sample from a prospective UC (or related disorder) patient before treatment and the total RNA quantity and quality is assessed as specified above in Example 1.
2. Total RNA is run in duplicate on three separate identical gene chip arrays, e.g., GeneChip Human Genome U133 Plus 2.0 arrays as follows:
   a. RNA amplification, target synthesis and labeling, chip hybridization, washing and staining are performed according to the manufacturer's protocol, e.g., Affymetrix, Santa Clara, CA.
   b. The GeneChips are scanned using, e.g., the GeneChip Scanner 3000.
   c. The data is analyzed with, e.g., GCOS 1.4 (GeneChip Operating System) using Affymetrix default analysis settings and global scaling as normalization method, with the trimmed mean target intensity of each array arbitrarily set to 500.
   d. The data quality is determined by correlating the data of each gene among the duplicates and across the three arrays.
      i. A correlation coefficient > 0.9 should be achieved.
   e. An average intensity value is calculated with a standard error representing the variability.
   f. The patient should respond to treatment with an anti-TNFα antibody (e.g., infliximab) if:
      i. The average intensity value is equal to or above X for each gene probe set (Table 2); or
      ii. The average intensity value for the five (or 20) gene panel is equal to or above X (Table 2).
   g. The patient should not respond to anti-TNFα antibody (e.g., infliximab) treatment if:
      i. The average intensity value is below Y for each gene probe set (Table 2); or
      ii. The average intensity value for the five (or 20) gene panel is below Y (Table 2).

**Table 1. Baseline Characteristics of the Study Cohorts**

| | **ACT1 Cohort** | |
|---|---|---|
| **Characteristics** | **Responders (n=12)** | **Non-responders (n=10)** |
| Male/Female | 6-Jun | 6-Apr |
| Median age at baseline (years) | 39.0 (29-70) | 51.5 (24-68) |
| Median weight at baseline (kg) | 75 (63.0-159.0) | 69.0 (46.0-102.0) |
| Median duration of disease at baseline (years) | 5.9 (1.6-42.1) | 5.7 (2.9-26.8) |
| Median C-reactive protein at baseline (mg/dL) | 0.7 (0.2-2.9) | 1.35 (0.2-6.8) |
| Concomitant medication at baseline | | |
| 5-Aminosalicylates | 1 | 1 |
| Corticosteroids | 8 | 6 |
| Azathioprine/6-Mercaptopurine | 2 | 3 |
| Corticosteroids + Immunosuppressants | 0 | 0 |
| Active smoking at baseline | 1 | 0 |

**Table 2: Baseline IFX probe set classifier**

| **Probe set ID** | **Ratio NR vs. R** | **Name (SEQ ID NO)** | **GenBank Acc No** |
|---|---|---|---|
| 205220_at | 11.1 | GPR109B (SEQ ID NO:1) | NM_006018 |
| 219434_at | 9.3 | TREM1 (SEQ ID NO:2) | NM_018643 |
| **230170_at** | **8.6** | **OSM** (SEQ ID NO:3) | **AI079327** |
| 213524_s_at | 7.7 | G0S2 (SEQ ID NO:4) | NM_015714 |
| **210773_s_at** | **7.1** | **FPRL1** (SEQ ID NO:5) | **U81501** |
| 236439_at | 6.5 | Unknown (SEQ ID NO:6) | AI733564 |
| 215671_at | 6.3 | PDE4B (SEQ ID NO:7) | AU144792 |
| 243296_at | 5.1 | PBEF1 (SEQ ID NO:8) | AA873350 |
| **228758_at** | **5.0** | **BCL6** (SEQ ID NO:9) | **AW264036** |
| **232555_at** | **4.6** | **CREB5** (SEQ ID NO:10) | **AI689210** |
| 39402_at | 4.3 | IL1B (SEQ ID NO:11) | M15330 |
| 1553297_a_at | 4.3 | CSF3R (SEQ ID NO:12) | NM_172313 |
| 204924_at | 4.0 | TLR2 (SEQ ID NO:13) | NM_003264 |
| **220088_at** | **3.7** | **C5AR1 (C5R1)** (SEQ ID NO:14) | **NM_001736** |
| 205237_at | 3.5 | FCN1 (SEQ ID NO:15) | NM_002003 |
| 1555643_s_at | 3.5 | LILRA5 (LIR9) (SEQ ID NO:16) | AF499918 |
| 211806_s_at | 3.3 | KCNJ15 (SEQ ID NO:17) | D87291 |
| 208438_s_at | 2.6 | FGR (SEQ ID NO:18) | NM_005248 |
| 211100_x_at | 2.5 | LILRA1 (SEQ ID NO:19) | U82278 |
| 215966_x_at | 2.4 | GK3P (GK) (SEQ ID NO:20) | AA292874 |

**Table 3: Characteristics of baseline IFX predictive signatures**

| | **20 probe set signature** | | **5 probe set signature** | |
|---|---|---|---|---|
| **Parameters** | **Test set** | **Validation set** | **Test set** | **Validation set** |
| R | 12 | 8 | 12 | 8 |
| Correct predictions | 11 | 7 | 11 | 7 |
| NR | 11 | 16 | 11 | 16 |
| Correct predictions | 11 | 11 | 10 | 11 |
| Sensitivity | 91.7% | 87.5% | 91.7% | 87.5% |
| Specificity | 100.0% | 68.8% | 90.9% | 68.8% |
| Overall accuracy | 95.7% | 75.0% | 91.3% | 75.0% |

**Table 3: Characteristics of baseline IFX predictive signatures**

| | **20 probe set signature** | | **5 probe set signature** | |
|---|---|---|---|---|
| **Parameters** | **Test set** | **Validation set** | **Test set** | **Validation set** |
| R | 12 | 8 | 12 | 8 |
| Correct predictions | 11 | 7 | 11 | 7 |
| NR | 11 | 16 | 11 | 16 |
| Correct predictions | 11 | 11 | 10 | 11 |
| Sensitivity | 91.7% | 87.5% | 91.7% | 87.5% |
| Specificity | 100.0% | 68.8% | 90.9% | 68.8% |
| Overall accuracy | 95.7% | 75.0% | 91.3% | 75.0% |

**Table 4 - Baseline differentially regulated genes comparing responders and nonresponders**

| | **Ratio** | | |
|---|---|---|---|
| **Probe set ID** | **NR vs. R** | **Name** | **GenBank Acc No** |
| 206924_at | 15.6 | IL11 | NM_000641 |
| 215078_at | 14.7 | SOD2 | AL050388 |
| 229947_at | 13.7 | Unknown | AI088609 |
| 207094_at | 13.5 | IL8RA | NM_000634 |
| 205220_at | 11.1 | GPR109B | NM_006018 |
| 232629_at | 9.9 | PROK2 | AF182069 |
| 211506_s_at | 9.8 | IL8 | AF043337 |
| 210119_at | 9.5 | KCNJ15 | U73191 |
| 1554997_a_at | 9.4 | PTGS2 | AY151286 |
| 219434_at | 9.3 | TREM1 | NM_018643 |
| 230170_at | 8.6 | OSM | AI079327 |
| 204748_at | 8.1 | PTGS2 | NM_000963 |
| 213524_s_at | 7.7 | G0S2 | NM_015714 |
| 210773_s_at | 7.1 | FPRL1 | U81501 |
| 214637_at | 7.0 | OSM | BG437034 |
| 205568_at | 6.7 | AQP9 | NM_020980 |
| 236439_at | 6.5 | Unknown | AI733564 |
| 206025_s_at | 6.3 | TNFAIP6 | AW188198 |
| 215671_at | 6.3 | PDE4B | AU144792 |
| 207008_at | 6.1 | IL8RB | NM_001557 |
| 203591_s_at | 5.9 | CSF3R | NM_000760 |
| 234644_x_at | 5.8 | Unknown | AK026079 |
| 206881_s_at | 5.8 | LILRA3 | NM_006865 |
| 229967_at | 5.8 | CKLFSF2 | AA778552 |
| 206522_at | 5.8 | MGAM | NM_004668 |
| 236495_at | 5.7 | Unknown | AI681868 |
| 206026_s_at | 5.6 | TNFAIP6 | NM_007115 |
| 205119_s_at | 5.5 | FPR1 | NM_002029 |
| 210873_x_at | 5.5 | APOBEC3A | U03891 |
| 203470_s_at | 5.4 | PLEK | AI433595 |
| 1554676_at | 5.3 | PRG1 | BC022313 |
| 204007_at | 5.3 | FCGR3A | J04162 |
| 243296_at | 5.1 | PBEF1 | AA873350 |
| 205681_at | 5.1 | BCL2A1 | NM_004049 |
| 210772_at | 5.1 | FPRL1 | M88107 |
| 228758_at | 5.0 | BCL6 | AW264036 |
| 212657_s_at | 4.9 | IL1RN | U65590 |
| 230746_s_at | 4.9 | STC1 | AW003173 |
| 207275_s_at | 4.8 | ACSL1 | NM_001995 |
| 205067_at | 4.7 | IL1B | NM_000576 |
| 232555_at | 4.6 | CREB5 | AI689210 |
| 204006_s_at | 4.6 | FCGR3A | NM_000570 |
| 212942_s_at | 4.6 | KIAA1199 | AB033025 |
| 210004_at | 4.6 | OLR1 | AF035776 |
| 216243_s_at | 4.5 | IL1RN | BE563442 |
| 204959_at | 4.5 | MNDA | NM_002432 |
| 39402_at | 4.3 | IL1B | M15330 |
| 1553297_a_at | 4.3 | CSF3R | NM_172313 |
| 201963_at | 4.3 | ACSL1 | NM_021122 |
| 212659_s_at | 4.0 | IL1RN | AW083357 |
| 204924_at | 4.0 | TLR2 | NM_003264 |
| 211163_s_at | 4.0 | TNFRSF10C | AF012536 |
| 204596_s_at | 3.9 | STC1 | U46768 |
| 229723_at | 3.9 | TAGAP | BF591040 |
| 204932_at | 3.8 | TNFRSF11B | BF433902 |
| 220088_at | 3.7 | C5R1 | NM_001736 |
| 205237_at | 3.5 | FCN1 | NM_002003 |
| 1555643_s_at | 3.5 | LIR9 | AF499918 |
| 229770_at | 3.4 | FLJ31978 | AI041543 |
| 214511_x_at | 3.4 | FCGR1A | L03419 |
| 210163_at | 3.4 | CXCL11 | AF030514 |
| 206222_at | 3.4 | TNFRSF10C | NM_003841 |
| 213425_at | 3.4 | WNT5A | AI968085 |
| 216950_s_at | 3.4 | FCGR1A | X14355 |
| 224941_at | 3.4 | PAPPA | BF107618 |
| 220404_at | 3.3 | GPR97 | NM_014076 |
| 206515_at | 3.3 | CYP4F3 | NM_000896 |
| 204933_s_at | 3.3 | TNFRSF11B | NM_002546 |
| 205118_at | 3.3 | FPR1 | M60626 |
| 211806_s_at | 3.3 | KCNJ15 | D87291 |
| 211122_s_at | 3.2 | CXCL11 | AF002985 |
| 226064_s_at | 3.1 | DGAT2 | AW469523 |
| 204597_x_at | 3.1 | STC1 | NM_003155 |
| 203140_at | 3.1 | BCL6 | NM_001706 |
| 204595_s_at | 3.1 | STC1 | AI300520 |
| 204422_s_at | 2.9 | FGF2 | NM_002006 |
| 225987_at | 2.8 | FLJ23153 | AA650281 |
| 205990_s_at | 2.7 | WNT5A | NM_003392 |
| 208438_s_at | 2.6 | FGR | NM_005248 |
| 201858_s_at | 2.6 | PRG1 | J03223 |
| 210484_s_at | 2.6 | TNFRSF10C | BC005043 |
| 232224_at | 2.6 | MASP1 | AI274095 |
| 208594_x_at | 2.6 | ILT8 | NM_024318 |
| 205100_at | 2.5 | GFPT2 | NM_005110 |
| 240862_at | 2.5 | RASGRP4 | AA923524 |
| 202388_at | 2.5 | RGS2 | NM_002923 |
| 219788_at | 2.5 | PILRA | NM_013439 |
| 211100_x_at | 2.5 | LILRA1 | U82278 |
| 224341_x_at | 2.4 | TLR4 | U93091 |
| 215966_x_at | 2.4 | GK | AA292874 |
| 201041_s_at | 2.4 | DUSP1 | NM_004417 |
| 215977_x_at | 2.4 | GK | X68285 |
| 205896_at | 2.3 | SLC22A4 | NM_003059 |
| 228501_at | 2.3 | GALNTL2 | BF055343 |
| 211133_x_at | 2.3 | LILRB3 | AF009643 |
| 217167_x_at | 2.2 | GK | AJ252550 |
| 211546_x_at | 2.2 | SNCA | L36674 |
| 210664_s_at | 2.1 | TFPI | AF021834 |
| 209960_at | 2.1 | HGF | X16323 |
| 219859_at | 2.0 | CLECSF9 | NM_014358 |
| 201315_x_at | 2.0 | IFITM3 | NM_006435 |
| 212281_s_at | -2.0 | MAC30 | BF038366 |
| 211541_s_at | -2.4 | DYRK1A | U52373 |
| 227491_at | -2.4 | Unknown | AA777752 |
| 235109_at | -2.8 | ZBED3 | AI887983 |
| 205523_at | -2.8 | HAPLN1 | U43328 |
| 232054_at | -5.3 | PCDH20 | AA040057 |
| 229831_at | -5.8 | CNTN3 | BE221817 |

### References

1. Rutgeerts P, Sandborn WJ, Feagan BG, et al. Infliximab for Induction and Maintenance Therapy for Ulcerative Colitis 2005. N Engl J Med 2005;353:2462-2476.
2. Geboes K, Riddell R, Ost A, et al. A reproducible grading scale for histological assessment of inflammation in ulcerative colitis. Gut 2000;47:404-409.
3. Bermejo S, Cabestany J. Adaptive soft k-nearest-neighbour classifiers. Pattern Recog 2000;33:1999-2005.
4. Liu Y, Shaw SK, Ma S, et al. Regulation of leukocyte transmigration: cell surface interactions and signaling events. J Immunol 2004;172:7-13.
5. Ben-Baruch A, Michiel DF, Oppenheim JJ. Signals and receptors involved in recruitment of inflammatory cells. J Biol Chem 1995;270:11703-11706.
6. Greenberg S, Grinstein S. Phagocytosis and innate immunity. Curr Opin Immunol 2002;14:136-145.
7. Izzo RS, Witkon K, Chen Al, et al. Interleukin-8 and neutrophils markers in colonic mucosa from patients with ulcerative colitis. Am J Gastroenterol 1992;87:1447-1452.
8. Al-Sadi R, Ye D, Dokladny K, Ma TY. Mechanism of IL-1{beta}-induced increase in intestinal epithelial tight junction permeability J Immunol. 2008;180:5653-5661.
9. Guo R-F, Riedemann NC, Ward PA. Role of C5a-C5ar interaction in sepsis. Shock 2004;21:1-7.
10. Hayashi F, Means TK, Luster AD. Toll-like receptors stimulate human neutrophil function. Blood 2003;102:2660-2669.
11. Frolova L, Drastich P, Rossmann P, et al. Expression of Toll-like Receptor 2 (TLR2), TLR4, and CD14 in Biopsy Samples of Patients With Inflammatory Bowel Diseases: Upregulated Expression of TLR2 in Terminal Ileum of Patients With Ulcerative Colitis, J Histochem Cytochem 2008;56:267-274.
12. Hart Al, Al-Hassi HO, Rigby RJ, et al. Characteristics of Intestinal Dendritic Cells in Inflammatory Bowel Diseases. Gastroenterology 2005;129:50-65.
13. Banks C, Bateman A, Payne R, et al. Chemokine expression in IBD. Mucosal chemokine expression is unselectively increased in both ulcerative colitis and Crohn's disease. J Pathol 2003;199:28-35.
14. Yamamoto T, Umegae S, Kitagawa T, et al. Systemic and local cytokine production in quiescent ulcerative colitis and its relationship to future relapse: a prospective pilot study. Inflamm Bowel Dis 2005;11:589-596.
15. Ramos CD, Heluy-Neto NE, Ribeiro RA, et al. Neutrophil migration induced by IL-8-activated mast cells is mediated by CINC-1. Cytokine 2003;21:214-223.
16. Ye BH, Cattoretti G, Shen Q, et al. The BCL-6 proto-oncogene controls germinal-centre formation and Th2-type. Inflamm Nat Genet 1997;16:161-70.
17. Kusam S, Toney LM, Sato H, et al. Inhibition of Th2 differentiation and GATA-3 expression by BCL-6. J Immunol 2003;170:2435-41.
18. Klein W, Tromm A, Griga T, et al. A polymorphism in the IL11 gene is associated with ulcerative colitis. Genes Immun 2002;3:494-496.
19. Balding J, Livingstone WJ, Conroy J, et al. Inflammatory bowel disease: the role of inflammatory cytokine gene polymorphisms. Med Inflam 2004;13:181-187.
20. Brun P, Castagliuolo I, Leo VD, et al. Increased intestinal permeability in obese mice: new evidence in the pathogenesis of nonalcoholic steatohepatitis Am J Physiol Gastrointest Liver Physiol. 2007;292:G518-G525.
21. Al-Sadi, RM, Ma TY. IL-1 b causes an increase in intestinal epithelial tight junction permeability. J. Immunol. 2007;178:4641-4649.

## Claims

1. A method for predicting the suitability of treatment with a target therapy for a gastrointestinal-related disorder in a subject, wherein the subject is a patient providing, the sample prior to administration of the therapy, and wherein the therapy is an anti-TNFα antibody, comprising:
a) preparing a sample of nucleic acids from a specimen obtained from the subject;
b) contacting the sample with a panel of nucleic acid segments consisting of SEQ ID NOS:1-20 to detect levels of the panel segments;
c) evaluating the sample against a reference standard of a responder to the therapy to determine the relative expression levels of all members from the group consisting of the nucleotide sequences corresponding to SEQ ID NOS:1-20 compared to the reference standard; and
d) correlating the relative expression levels of the sample and the reference standard with the suitability of treatment with the target therapy for the gastrointestinal-related disorder.

2. The method of claim 1, wherein the anti-TNFα antibody is infliximab.

3. The method of claim 2, wherein the gastrointestinal-related disorder is ulcerative colitis.

4. The method of claim 3, wherein the reference standard is from a colon biopsy from a responder to the target therapy.

5. The method of claim 1, wherein the panel is an array of nucleic acid segments.

6. The method of claim 1, wherein the sample comprises a colon biopsy sample or peripheral blood cells.

7. A method according to claim 1 which is an array-based testing method for predicting the suitability of treatment with a target therapy for a gastrointestinal-related disorder in a patient, comprising:
a) preparing a mixture of nucleic acids from a specimen obtained from the patient;
b) labeling said specimen nucleic acids with a detectable marker to form a sample;
c) contacting the sample with an array comprising a plurality of nucleic acid segments, wherein each nucleic acid segment is immobilized to a discrete and known address on a substrate surface of the array, wherein a gastrointestinal-related gene panel consisting of the nucleotide sequences corresponding to SEQ ID NOS: 1-20 are identified as features of the array by address, and wherein said array further comprises at least one calibration nucleic acid at a known address on the substrate;
d) determining the degree of binding of the specimen nucleic acids to the nucleic acid segments; and
e) comparing the degree of binding to the reference standard to enable an assessment of the suitability of treatment.

8. The method of claim 7, wherein the comparing step comprises evaluating the sample against a reference standard to determine the magnitude of change in the amounts of the nucleotide sequences corresponding to SEQ ID NOS:1-20.

9. The method of claim 8, wherein:
i) the gastrointestinal-related disorder is ulcerative colitis and the gastrointestinal-related gene panel is an ulcerative colitis-related gene panel;
ii) the target therapy is an anti-TNFα antibody;
iii) the specimen is from a colon biopsy of a patient selected from the group consisting of patients suspected of having ulcerative colitis and patients diagnosed with ulcerative colitis not undergoing treatment;
iv) the specimen is from a source selected from the group consisting of a patient providing the specimen prior to administration of a therapy, a patient having a similar disease or condition treated with a placebo, and a sample from a biobank;
v) the specimen comprises a colon biopsy sample or peripheral blood cells; or
vi) the comparing the degree of binding step further comprises a stringent test of the similarity of feature intensity changes of the array of the ulcerative colitis-related gene panel.

10. A reagent for testing the suitability of an anti-TNFα antibody for a gastrointestinal-related disorder in a cell or subject prior to administration of the anti-TNFα antibody, comprising oligonucleotides comprising at least 15 nucleotides comprising or complementary to a nucleotide sequence of each of the nucleotide sequences corresponding to SEQ ID NOS: 1-20,

11. The reagent of claim 10, wherein the gastrointestinal-related disorder is ulcerative colitis and optionally wherein the anti-TNFα antibody is infliximab.

12. A method of testing the suitability of an anti-TNFα antibody for a gastrointestinal-related disorder in a patient sample from a patient prior to administration of the anti-TNFα antibody, comprising contacting the patient sample with the reagent of claim 10 and comparing the levels of at least a portion of each of the genes of the nucleotide sequences corresponding to SEQ ID NOS: 1-20 to a reference standard, wherein the reference standard is of a responder to the anti-TNFα antibody.

13. The method of claim 12:
i) wherein the testing is done by RT-PCR or
ii) wherein the anti-TNFα antibody is infliximab.

14. A method of testing the effectiveness of an anti-TNFα antibody for ulcerative colitis, comprising:
a) contacting a sample from a patient being treated for ulcerative colitis with the reagent of claim 10;
b) measuring levels of the 20 members; and
c) correlating the levels of the 20 members with the effectiveness of the anti-TNFα antibody by comparing the levels to the levels in a reference standard, wherein the reference standard is of a responder to the anti-TNFα antibody.

15. The method of claim 14, wherein the anti-TNFα antibody is infliximab.

## Patentansprüche

1. Verfahren zum Vorhersagen der Eignung einer Behandlung mit einer Zieltherapie für eine mit dem Gastrointestinaltrakt zusammenhängende Störung bei einem Individuum, wobei das Individuum ein Patient ist, der die Probe vor der Verabreichung der Therapie bereitstellt, und wobei es sich bei der Therapie um einen Anti-TNFα-Antikörper handelt, umfassend:
a) Herstellen einer Probe von Nukleinsäuren von einer von dem Individuum erhaltenen Einzelprobe,
b) In-Kontakt-Bringen der Probe mit einem Panel von Nukleinsäuresegmenten, die aus den SEQ ID NO: 1-20 bestehen, um Spiegel der Panel-Segmente zu bestimmen,
c) Untersuchen der Probe gegenüber einem Bezugsstandard von einem Responder auf die Therapie, um die relativen Expressionsspiegel aller Mitglieder aus der Gruppe, die aus den Nukleotidsequenzen besteht, die den SEQ ID NO: 1-20 entsprechen, im Vergleich zu dem Bezugsstandard zu bestimmen, und
d) Korrelieren der relativen Expressionsspiegel der Probe und des Bezugsstandards mit der Eignung einer Behandlung mit der Zieltherapie für eine mit dem Gastrointestinaltrakt zusammenhängende Störung.

2. Verfahren nach Anspruch 1, wobei der Anti-TNFα-Antikörper Infliximab ist.

3. Verfahren nach Anspruch 2, wobei die mit dem Gastrointestinaltrakt zusammenhängende Störung Colitis ulcerosa ist.

4. Verfahren nach Anspruch 3, wobei der Bezugsstandard von einer Kolonbiopsie von einem Responder auf die Zieltherapie herrührt.

5. Verfahren nach Anspruch 1, wobei das Panel ein Array von Nukleinsäuresegmenten ist.

6. Verfahren nach Anspruch 1, wobei die Probe eine Kolonbiopsieprobe oder periphere Blutzellen umfasst.

7. Verfahren nach Anspruch 1, das ein auf einem Array basierendes Testverfahren zum Vorhersagen der Eignung einer Behandlung mit einer Zieltherapie für eine mit dem Gastrointestinaltrakt zusammenhängende Störung bei einem Patienten ist, umfassend:
a) Herstellen eines Gemischs von Nukleinsäuren von einer von dem Patienten erhaltenen Einzelprobe,
b) Markieren der Einzelproben-Nukleinsäuren mit einem nachweisbaren Marker, um eine Probe herzustellen,
c) In-Kontakt-Bringen der Probe mit einem Array, der eine Mehrzahl von Nukleinsäuresegmenten umfasst, wobei jedes Nukleinsäuresegment an einer separaten und bekannten Adresse auf einer Substratoberfläche des Arrays immobilisiert ist, wobei ein mit dem Gastrointestinaltrakt zusammenhängendes Gen-Panel, das aus den Nukleotidsequenzen besteht, die den SEQ ID NO: 1-20 entsprechen, als Merkmale des Arrays anhand der Adresse identifiziert wird und wobei der Array weiterhin mindestens eine Eichnukleinsäure an einer bekannten Adresse auf dem Substrat umfasst,
d) Bestimmen des Grads der Bindung der Einzelproben-Nukleinsäuren an die Nukleinsäuresegmente und
e) Vergleichen des Grads der Bindung an den Bezugsstandard, um eine Einschätzung der Eignung einer Behandlung zu ermöglichen.

8. Verfahren nach Anspruch 7, wobei der Vergleichsschritt das Untersuchen der Probe gegenüber einem Bezugsstandard umfasst, um das Ausmaß einer Veränderung in den Mengen der Nukleotidsequenzen, die den SEQ ID NO: 1-20 entsprechen, zu bestimmen.

9. Verfahren nach Anspruch 8, wobei:
i) die mit dem Gastrointestinaltrakt zusammenhängende Störung Colitis ulcerosa ist und das mit dem Gastrointestinaltrakt zusammenhängende Gen-Panel ein mit Colitis ulcerosa zusammenhängendes Gen-Panel ist,
ii) die Zieltherapie ein Anti-TNFα-Antikörper ist,
iii) die Einzelprobe von einer Kolonbiopsie eines Patienten herrührt, der aus einer Gruppe ausgewählt ist, bestehend aus Patienten, von denen vermutet wird, das sie Colitis ulcerosa haben, und Patienten, bei denen Colitis ulcerosa diagnostiziert wurde, die keiner Behandlung unterliegen,
iv) die Einzelprobe aus einer Quelle stammt, ausgewählt aus der Gruppe, bestehend aus einem Patienten, der die Einzelprobe vor der Verabreichung einer Therapie bereitstellt, einem Patienten mit einer ähnlichen Krankheit oder einem ähnlichen Zustand, der mit einem Placebo behandelt wird, und einer Plasmaprobe aus einer Biobank,
v) die Einzelprobe eine Kolonbiopsie oder periphere Blutzellen umfasst oder
vi) der Schritt des Vergleichens des Grads der Bindung zudem einen stringenten Test der Ähnlichkeit von Merkmalsintensitätsveränderungen des Arrays des mit Colitis ulcerosa zusammenhängenden Gen-Panels umfasst.

10. Reagenz zum Testen der Eignung eines Anti-TNFα-Antikörpers für eine mit dem Gastrointestinaltrakt zusammenhängende Störung in einer Zelle oder einem Individuum vor der Verabreichung des Anti-TNFα-Antikörpers, umfassend Oligonukleotide, die mindestens 15 Nukleotide umfassen, die eine Nukleotidsequenz von jeder der Nukleotidsequenzen, die den SEQ ID NO: 1-20 entsprechen, umfassen oder komplementär dazu sind.

11. Reagenz nach Anspruch 10, wobei die mit dem Gastrointestinaltrakt zusammenhängende Störung Colitis ulcerosa ist und wobei gegebenenfalls der Anti-TNFα-Antikörper Infliximab ist.

12. Verfahren zum Testen der Eignung eines Anti-TNFα-Antikörpers bei einer mit dem Gastrointestinaltrakt zusammenhängenden Störung in einer Patientenprobe von einem Patienten vor der Verabreichung des Anti-TNFα-Antikörpers, umfassend das In-Kontakt-Bringen der Patientenprobe mit einem Reagenz nach Anspruch 10 und Vergleichen der Spiegel von mindestens einem Anteil von jedem der Gene der Nukleotidsequenzen, die den SEQ ID NO: 1-20 entsprechen, mit einem Bezugsstandard, wobei der Bezugsstandard von einem Responder auf den Anti-TNFα-Antikörper herrührt.

13. Verfahren nach Anspruch 12:
i) wobei das Testen mittels RT-PCR durchgeführt wird oder
ii) wobei der Anti-TNFα-Antikörper Infliximab ist.

14. Verfahren zum Testen der Wirksamkeit eines Anti-TNFα-Antikörpers bei Colitis ulcerosa, umfassend:
a) In-Kontakt-Bringen einer Probe von einem Patienten, der wegen Colitis ulcerosa behandelt wird, mit dem Reagenz nach Anspruch 10,
b) Messen der Spiegel der 20 Mitglieder und
c) Korrelieren der Spiegel der 20 Mitglieder mit der Wirksamkeit des Anti-TNFα-Antikörpers durch Vergleichen der Spiegel mit den Spiegeln in einem Bezugsstandard, wobei der Bezugsstandard von einem Responder auf den Anti-TNFα-Antikörper herrührt.

15. Verfahren nach Anspruch 14, wobei der Anti-TNFα-Antikörper Infliximab ist.

## Revendications

1. Procédé pour prédire la convenance d'un traitement utilisant une thérapie ciblée pour un trouble associé au tractus gastro-intestinal chez un sujet, le sujet étant un patient fournissant l'échantillon avant administration de la thérapie, et dans lequel la thérapie est un anticorps anti-TNFα, comprenant :
a) la préparation d'un échantillon d'acides nucléiques provenant d'un spécimen obtenu du sujet ;
b) la mise en contact de l'échantillon avec un panel de segments d'acides nucléiques consistant en SEQ ID NO:1-20 pour détecter le niveau des segments du panel ;
c) l'évaluation de l'échantillon par comparaison avec un étalon de référence d'un répondant à la thérapie, pour déterminer les niveaux d'expression relative de tous les membres du groupe consistant en les séquences nucléotidiques correspondant à SEQ ID NO:1-20 par comparaison avec l'étalon de référence ; et
d) la corrélation des niveaux d'expression relative de l'échantillon et de l'étalon de référence avec la convenance du traitement avec la thérapie ciblée pour le trouble associé au tractus gastro-intestinal.

2. Procédé selon la revendication 1, dans lequel l'anticorps anti-TNFα est l'infliximab.

3. Procédé selon la revendication 2, dans lequel le trouble associé au tractus gastro-intestinal est la rectocolite hémorragique.

4. Procédé selon la revendication 3, dans lequel l'étalon de référence provient d'une biopsie du côlon obtenue d'un répondant à la thérapie cible.

5. Procédé selon la revendication 1, dans lequel le panel est une biopuce de segments d'acides nucléiques.

6. Procédé selon la revendication 1, dans lequel l'échantillon comprend un échantillon de biopsie du côlon ou des cellules du sang périphérique.

7. Procédé selon la revendication 1, qui est une méthode d'essai se fondant sur une biopuce pour prédire la convenance d'un traitement avec une thérapie cible pour un trouble associé au tractus gastro-intestinal chez un patient, comprenant :
a) la préparation d'un mélange d'acides nucléiques à partir d'un spécimen obtenu du patient ;
b) le marquage desdits acides nucléiques du spécimen avec un marqueur détectable pour former un échantillon ;
c) la mise en contact de l'échantillon avec une biopuce comprenant une pluralité de segments d'acides nucléiques, chaque segment d'acide nucléique étant immobilisé sur une adresse discrète et connue d'une surface de substrat de la biopuce, le panel de gènes associés au tractus gastro-intestinal consistant en les séquences nucléotidiques correspondant à SEQ ID NO:1-20 étant identifiés comme des caractéristiques de la biopuce par adresse, et la dite biopuce comprenant en outre au moins un acide nucléique d'étalonnage à une adresse connue sur le substrat ;
d) la détermination du degré de liaison des acides nucléiques du spécimen aux segments d'acides nucléiques ; et
e) la comparaison du degré de liaison à l'étalon de référence pour permettre une évaluation de la convenance du traitement.

8. Procédé selon la revendication 7, dans lequel l'étape de comparaison comprend l'évaluation de l'échantillon par comparaison avec un étalon de référence pour déterminer l'importance du changement des quantités des séquences nucléotidiques correspondant à SEQ ID NO:1-20.

9. Procédé selon la revendication 8, dans lequel :
i) le trouble associé au tractus gastro-intestinal est la rectocolite hémorragique, et le panel de gènes associés au tractus gastro-intestinal est un panel de gènes associés à la rectocolite hémorragique ;
ii) la thérapie ciblée est un anticorps anti-TNFα ;
iii) le spécimen provient d'une biopsie du côlon d'un patient choisi dans le groupe consistant en les patients suspectés de présenter une rectocolite hémorragique et les patients diagnostiqués avec une rectocolite hémorragique ne subissant aucun traitement ;
iv) le spécimen provient d'une source choisie dans le groupe consistant en un patient fournissant le spécimen avant administration d'une thérapie, un patient ayant une maladie ou un état pathologique similaire, traité avec un placébo, et un échantillon provenant d'une biobanque ;
v) le spécimen comprend un échantillon de biopsies du côlon ou des cellules du sang périphérique ; ou
vi) l'étape de comparaison du degré de liaison comprend en outre un test stringent portant sur la similarité des changements d'intensité de caractéristique de la puce du panel de gènes associés à la rectocolite hémorragique.

10. Réactif pour tester la convenance d'un anticorps anti-TNFα pour ce qui est d'un trouble associé au tractus gastro-intestinal dans une cellule ou un sujet avant administration de l'anticorps anti-TNFα, comprenant des oligonucléotides comprenant au moins 15 nucléotides comprenant une séquence nucléotidique de chacune des séquences nucléotidiques correspondant à SEQ ID NO:1-20, ou qui leur sont complémentaires.

11. Réactif selon la revendication 10, dans lequel le trouble associé au tractus gastro-intestinal est la rectocolite hémorragique, et en option dans lequel l'anticorps anti-TNFα est l'infliximab.

12. Procédé pour tester la convenance d'un anticorps anti-TNFa pour ce qui est d'un trouble associé au tractus gastro-intestinal dans un échantillon d'un patient provenant d'un patient avant administration de l'anticorps anti-TNFα, comprenant la mise en contact de l'échantillon du patient avec le réactif de la revendication 10, et la comparaison des niveaux d'au moins une portion de chacun des gènes des séquences nucléotidiques correspondant à SEQ ID NO:1-20 à un étalon de référence, l'étalon de référence provenant d'un répondant à l'anticorps anti-TNFα.

13. Procédé selon la revendication 12 :
i) dans lequel l'essai est effectué par RN-PCR, ou
ii) dans lequel l'anticorps anti-TNFα est l'infliximab.

14. Procédé pour tester l'efficacité d'un anticorps anti-TNFα pour ce qui est d'une rectocolite hémorragique, comprenant :
a) la mise en contact d'un échantillon provenant d'un patient traité pour une rectocolite hémorragique avec le réactif de la revendication 10 ;
b) la mesure du niveau des 20 membres ; et
c) la corrélation du niveau des 20 membres avec l'efficacité de l'anticorps anti-TNFα par comparaison des niveaux aux niveaux dans un étalon de référence, l'étalon de référence provenant d'un répondant à l'anticorps anti-TNFα.

15. Procédé selon la revendication 14, dans lequel l'anticorps anti-TNFα est l'infliximab.
